(19) Europäisches Patentamt
European Patent Office
Office européen des brevets

(11) **EP 3 033 648 B1**

(12) **EUROPÄISCHE PATENTSCHRIFT**

(45) Veröffentlichungstag und Bekanntmachung des Hinweises auf die Patenterteilung:
**10.03.2021 Patentblatt 2021/10**

(21) Anmeldenummer: **14747037.1**

(22) Anmeldetag: **04.08.2014**

(51) Int Cl.:
*G02B 27/00* (2006.01)   *G02C 7/04* (2006.01)
*A61F 2/16* (2006.01)

(86) Internationale Anmeldenummer:
**PCT/EP2014/066699**

(87) Internationale Veröffentlichungsnummer:
**WO 2015/022216 (19.02.2015 Gazette 2015/07)**

(54) **AUGENLINSE, INSBESONDERE INTRAOKULARLINSE, MIT EINEM TORISCH BRECHENDEN OBERFLÄCHENPROFIL UND EINER HELIXWINDUNG ALS OBERFLÄCHENSTRUKTUR AUF EINEM OPTISCHEN TEIL**

EYE LENS, IN PARTICULAR INTRAOCULAR LENS, HAVING A TORIC REFRACTIVE SURFACE PROFILE AND A HELIX WINDING AS A SURFACE STRUCTURE ON AN OPTICAL PART

LENTILLE, EN PARTICULIER LENTILLE INTRAOCULAIRE, PRÉSENTANT UN PROFIL DE SURFACE À RÉFRACTION TORIQUE ET UN ENROULEMENT HÉLICOÏDAL EN TANT QUE STRUCTURE DE SURFACE SUR UNE PARTIE OPTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.08.2013 DE 102013215984**

(43) Veröffentlichungstag der Anmeldung:
**22.06.2016 Patentblatt 2016/25**

(73) Patentinhaber: **Carl Zeiss Meditec AG**
**07745 Jena (DE)**

(72) Erfinder: **GERLACH, Mario**
**16548 Glienicke-Nordbahn (DE)**

(74) Vertreter: **Hofstetter, Schurack & Partner**
**Patent- und Rechtsanwaltskanzlei**
**PartG mbB**
**Balanstrasse 57**
**81541 München (DE)**

(56) Entgegenhaltungen:
EP-A2- 0 949 529       WO-A1-2011/102719
WO-A1-2012/085917     WO-A2-2010/083546
DE-A1-102011 101 899   DE-A1-102011 114 752

**Beschreibung**

Die Erfindung betrifft eine Augenlinse mit einem optischen Teil, der eine in Richtung einer optischen Hauptachse der Augenlinse betrachtet optische Vorderseite und eine optische Rückseite aufweist. Auf zumindest einer dieser beiden Seiten ist eine Helixwindung als Oberflächenstruktur ausgebildet, durch welche eine Brechkraft der Linse in Umlaufrichtung um die Hauptachse wertmäßig variiert.

Stand der Technik

Linsen, die eine derartige azimutale Brechkraftvariation durch eine entsprechend gewundene und somit spiralförmig beziehungsweise helixförmig gestaltete Oberflächenkontur aufweist, sind aus der DE 10 2011 101 899 A1 und der DE 10 2011 114 752 A1 bekannt. Durch derartige Ausgestaltungen mit einer azimutal veränderlichen Brechkraftverteilung wird eine Schärfentiefe der Linse verbessert beziehungsweise erweitert.

Aus der WO 2011/102719 A1 ist eine Intraokularlinse bekannt, die als Struktur auf einer Oberfläche eines optischen Teils eine Helixwindung oder eine einen Astigmatismus korrigierende optische Oberfläche aufweisen kann.

Das menschliche Auge kann aufgrund seines relativ komplexen Aufbaus und somit auch insbesondere der zur optischen Abbildung vorgesehenen Teile und/oder anderweitiger Einflussfaktoren mit unterschiedlichsten Sehfehlern behaftet sein. Diese können individuell in der Stärke unterschiedlich ausgeprägt sein, andererseits auch eine Mehrzahl unterschiedliche Sehfehler an einem Auge vorliegen.

Um diese Sehfehlerkomplexität zumindest wesentlich verbessern zu können, ist es stetiges Bestreben, Augenlinsen dahingehend weiterzuentwickeln. Gerade bei Intraokularlinsen sind in dem Zusammenhang auch bezüglich der Handhabung bei einem operativen Eingriff die Kleinschnitttauglichkeit und somit das Einführen einer entsprechend gefalteten Intraokularlinse durch einen möglichst kleinen Schnitt im Auge, als auch die Kompaktheit einer derartigen Linse ebenso wesentliche Aspekte, die zu berücksichtigen sind.

Unter Berücksichtigung all dieser Anforderungen müssen somit neben einer Vielzahl und teils gegensätzlichen optischen Anforderungen auch entsprechende Handhabbarkeitsanforderungen erfüllt werden.

Ausgehend von den bekannten Linsen, bei denen eine Verbesserung der Schärfentiefe durch diese spiralförmige beziehungsweise helixförmige Windung einer Oberflächengestaltung vorgesehen ist, sollen auch anderweitige Sehfehler korrigiert werden bei gleichzeitiger Erreichung der Kleinschnittauglichkeit.

Darstellung der Erfindung

Es ist Aufgabe der vorliegenden Erfindung, eine kleinschnitttaugliche Augenlinse mit einem möglichst großen Schärfentiefenbereich zu schaffen, bei dem darüber hinaus auch eine Korrektur von anderweitigen Sehfehlern ermöglicht ist.

Diese Aufgabe wird durch eine Augenlinse, wie sie durch die Merkmale des Anspruchs 1 ausgebildet ist, gelöst.

Eine erfindungsgemäße Augenlinse umfasst einen optischen Teil, mit welchem die Abbildungseigenschaften der Augenlinse charakterisiert sind. Dieser optische Teil weist eine in Richtung einer optischen Hauptachse dieser Augenlinse betrachtet optische erste Seite, die eine Vorderseite oder eine Rückseite sein kann, und eine der ersten Seite am optischen Teil axial gegenüberliegende zweite optische Seite auf, die abhängig von der ersten Seite dann eine Rückseite oder Vorderseite sein kann. Die optische Hauptachse steht senkrecht auf einer axial zwischen der ersten und der zweiten Seite, insbesondere der Vorderseite und der Rückseite, liegenden Ebene der Augenlinse, insbesondere des optischen Teils, so dass die Seiten gegenüberliegend zu dieser Ebene entlang der Hauptachse betrachtet angeordnet sind. Zumindest auf einer der beiden Seiten und somit zumindest auf der Vorderseite oder der Rückseite, ist eine zur optischen Abbildungseigenschaft der Augenlinse beitragende Helixwindung als Oberflächenstruktur ausgebildet. Durch eine derartige Helixwindung wird eine kontinuierliche Variation einer Brechkraft der Augenlinse in azimutaler Richtung und somit in Umlaufrichtung um die optische Hauptachse wertmäßig erreicht.

Ein wesentlicher Gedanke der Erfindung ist darin zu sehen, dass zusätzlich zu dieser Helixwindung als Oberflächenstruktur auf zumindest einer Seite des optischen Teils ein zur optischen Abbildungseigenschaft beitragendes torisch brechendes Oberflächenprofil ausgebildet ist.

Durch eine derartige Ausgestaltung einer Augenlinse wird neben einem erweiterten Fokusbereich und somit einer Vergrößerung des Schärfentiefenbereichs zusätzlich auch eine Korrektur eines Astigmatismus und Aberrationen höherer Ordnung erreicht. Darüber hinaus wird auch die Abhängigkeit der Abbildungsleistung der erfindungsgemäßen Linse gegenüber der Rotation der erfindungsgemäßen Linse zur Achse des kornealen Astigmatismus reduziert.

Dieses torisch brechende Oberflächenprofil kann um weitere radiale und azimutale Brechkraftkomponenten ergänzt werden, um eine Korrektur von Wellenfrontfehlern höherer Ordnung zu ermöglichen.

Der Sehfehler Astigmatismus ist dadurch charakterisiert, dass sich die wirksamen Brechkräfte zweier unterschiedlicher Azimuten unterscheiden und dadurch eine punktförmige Abbildung eines Objektpunkts verhindert wird. In der Regel stehen die beiden Azimute senkrecht zueinander und stellen dadurch Hauptmeridiane dar. Die primäre Quelle für den Astigmatismus des menschlichen Auges ist eine gestörte Rotationssymmetrie der menschlichen Hornhaut. Hierbei unterscheiden sich in zwei senkrecht zueinander stehenden Richtungen die Krümmungsradien. Gerade im Fall einer Kataraktoperation kann die Korrektur des kornealen Astigmatismus von einer Intraokularlinse übernommen werden und dem Patienten ein brillenfreies Sehen ermöglicht werden.

Insbesondere stehen Hauptmeridiane des torisch brechenden Oberflächenprofils senkrecht aufeinander in sind somit azimutal entsprechend 90° versetzt zueinander.

Die Augenlinse ist eine Intraokularlinse.

Es kann vorgesehen sein, dass das torisch brechende Oberflächenprofil und die Helixwindung auf einer gemeinsamen Seite ausgebildet und überlagert sind. Dies kann die Vorderseite oder aber auch die Rückseite sein. Durch eine derartige Ausgestaltung kann die Lage der beiden unterschiedlichen separaten Oberflächenstrukturen beziehungsweise Oberflächenprofile sehr genau erreicht werden.

Es kann auch vorgesehen sein, dass das torisch brechende Oberflächenprofil auf einer Seite und die Helixwindung auf der anderen Seite ausgebildet sind. Dabei kann es vorgesehen sein, dass die Helixwindung auf der Vorderseite und das torisch brechende Oberflächenprofil auf der Rückseite ausgebildet sind oder das torisch brechende Oberflächenprofil auf der Vorderseite und die Helixwindung auf der Rückseite ausgebildet sind. Bei einer derartigen Ausgestaltung sind die individuellen separaten Oberflächenstrukturen beziehungsweise Oberflächenprofile in ihrer Kontur sehr genau herstellbar, so dass die Einzelstrukturen dann sehr präzise Abbildungseigenschaften ermöglichen.

Es kann auch vorgesehen sein, dass sowohl auf der Vorderseite als auch auf der Rückseite eine Helixwindung ausgebildet ist. Diese beiden Helixwindungen sind dann so ausgebildet, dass sie in Summe die gesamte azimutale Brechkraft der Helixwindung als Beitrag zur gesamten Brechkraft der Augenlinse bilden. Im Vergleich zu einer Aufbringung der Helixwindung nur auf eine der Seiten wird bei dieser Aufteilung somit die optische Wirkung bezüglich der Brechkraftverteilung auf die Seiten aufgeteilt.

Entsprechendes kann zusätzlich oder anstatt dazu auch mit dem torisch brechenden Oberflächenprofil vorgesehen sein. Auch hier kann ein derartiges Profil auf der Vorderseite und der Rückseite ausgebildet sein. Die Summe der Teilbrechkräfte dieser torischen Profile ergibt dann den gesamten Brechkraftanteil des torisch brechenden Oberflächenprofils der Augenlinse, der zur Gesamtbrechkraft beiträgt.

Vorzugsweise ist vorgesehen, dass die Helixwindung als Oberflächenstruktur eine einmal vollständig um die optische Hauptachse der Augenlinse umlaufende Windung ist, und zwischen einem Windungsanfang und einem Windungsende ein sprungartiger Übergang ausgebildet ist. Durch einen derartigen möglichst diskreten Übergang, der in azimutaler Richtung über einen möglichst kleinen Winkelbereich, insbesondere kleiner 20°, insbesondere kleiner 10°, ausgebildet ist, wird die sich verändernde, insbesondere kontinuierlich verändernde, insbesondere vom Windungsanfang zum Windungsende kontinuierlich erhöhende, Brechkraftverteilung in azimutaler Richtung begünstigt. Durch einen möglichst azimutal kleinen Übergang wird die optische Abbildungseigenschaft der Linse verbessert.

Vorzugsweise ist vorgesehen, dass diejenige Seite des optischen Teils, auf welcher zumindest die Helixwindung ausgebildet ist, einen Scheitel aufweist und eine Differenz von einer Scheitelkrümmung am Windungsanfang und einer Scheitelkrümmung am Windungsende zwischen 0,001 mm$^{-1}$ und 0,16 mm$^{-1}$ beträgt. Gerade durch eine derartige Ausgestaltung der Differenz der Scheitelkrümmungen links- und rechtsseitig von diesem Sprung beziehungsweise diesem Übergang ermöglicht es, Brechkraftadditionen von 0,5 Dioptrien bis 20 Dioptrien für einen Bereich des Brechungsindex des Materials des optischen Teils zwischen 1,43 und 1,6 auszubilden. Vorzugsweise beträgt die Differenz dieser Scheitelkrümmungen zwischen 0,1 mm$^{-1}$ und 0,16 mm$^{-1}$, insbesondere zwischen, 0,5 mm$^{-1}$ und 0,16 mm$^{-1}$, insbesondere zwischen 0,8 mm$^{-1}$ und 0,16 mm$^{-1}$, insbesondere zwischen 0,8 mm$^{-1}$ und 0,12 mm$^{-1}$. Grundsätzlich sind in dem oben genannten größten Intervall jedoch auch alle möglichen Teilintervalle als offenbart anzusehen.

Vorzugsweise ist vorgesehen, dass diejenige Seite des optischen Teils, auf welcher zumindest die Helixwindung ausgebildet ist, einen Scheitel aufweist und eine Differenz von einer Scheitelkrümmung am Windungsanfang und einer Scheitelkrümmung am Windungsende unabhängig von einer Brechkraft am Windungsanfang ist. Dies bedeutet somit in vorteilhafter Ausführung, dass die Krümmungsdifferenz unabhängig von der Brechkraftstufe beziehungsweise der Zielbrechkraft der Augenlinse ist. Die Zielbrechkraft ist in dem Zusammenhang der niedrigste Wert der Brechkraft der Helixwindung, der somit insbesondere an der Stelle auftritt, an der diese Helixwindung die geringste Scheitelkrümmung aufweist. Dies ist insbesondere der Windungsanfang.

In einer weiteren vorteilhaften Ausführung ist vorgesehen, dass die Differenz der Scheitelkrümmungen unabhängig von der Brechkraft am Windungsanfang und somit vorzugsweise bei der Zielbrechkraft der Augenlinse, ist.

Insbesondere ist die Differenz der Scheitelkrümmungen abhängig von der Brechkraftaddition und/oder der Ausgestaltung der jeweils komplementären Linsenoberflächen beziehungsweise der Vorderseite und der Rückseite, sowie den Brechungsindizes der optischen Medien, und somit des Polymermaterials des optischen Teils und/oder dem Immersionsmedium im Auge, welches bekanntermaßen einen Brechungsindex von 1,336 aufweist.

In einer weiteren vorteilhaften Ausführung ist vorgesehen, dass die Differenz der Scheitelkrümmungen unabhängig von der Brechkraft am Windungsanfang und somit insbesondere der Zielbrechkraft, konstant ist.

Vorzugsweise ist vorgesehen, dass die Differenz der Scheitelkrümmungen abhängig von der Differenz der Brechkräfte am Windungsanfang und am Windungsende variiert. Bevorzugt ist hier vorgesehen, dass die Differenz der Scheitelkrümmungen für eine Differenz der Brechkräfte zwischen 1 Dioptrien und 10 Dioptrien zwischen 0,015 mm$^{-1}$ und 0,085 mm$^{-1}$ variiert.

Insbesondere kann vorgesehen sein, dass der konstante Wert der Differenz der Scheitelkrümmungen für eine Zielbrechkraft von -10 Dioptrien bis 40 Dioptrien zwischen 0,08 und 0,082 mm$^{-1}$ für eine Brechkraftdifferenz von 10 Dioptrien beträgt. In einem weiteren Beispiel kann vorgesehen sein, dass die Differenz der Scheitelkrümmungen für eine Zielbrechkraft von - 10 bis 40 Dioptrien zwischen 0,04 und 0,041 mm$^{-1}$ für eine Brechkraftdifferenz von 5 Dioptrien beträgt. Bei einem weiteren Ausführungsbeispiel kann vorgesehen sein, dass die Differenz der Scheitelkrümmungen für einen Wert der Zielbrechkraft zwischen -10 und 40 Dioptrien dann 0,02 bis 0,021 mm$^{-1}$ bei einer Brechkraftdifferenz von 2,5 Dioptrien beträgt. In einem weiteren Beispiel kann vorgesehen sein, dass die Differenz der Scheitelkrümmungen zwischen 0,007 und 0,009 mm$^{-1}$ für eine Zielbrechkraft zwischen -10 und 40 Dioptrien und einer Brechkraftdifferenz von 1 Dioptrien beträgt. Diese Angaben sind insbesondere für ein Material des optischen Teils der Linse auftretend, dass einen Brechungsindex von 1,46 aufweist.

Für die Bestimmung der Differenz der Scheitelkrümmungen kann insbesondere als ein Beispiel nachstehend aufgelistete Formel 1 zugrunde gelegt werden:

$$\Delta K = K1 - K2 = \Delta P \; \frac{n_{IOL}\,rk}{(n_{IOL}-n_{Med})(ct(n_{med}-n_{IOL})+n_{IOL}r_k)} \qquad (1)$$

mit

$\Delta K$ =            Differenz der Krümmungen
K1, K2 =      Krümmungen unmittelbar rechtseitig und linksseitig vom Sprung
$\Delta P$ =            Brechkraftaddition (Brechkraftdifferenz)
$n_{med}$ =          Brechungsindex des Immersionsmediums
$n_{IOL}$ =          Brechungsindex der Linse
ct =             Mittendicke der Linse
$r_k$ =             Krümmungsradius der gegenüberliegenden Linsenfläche

Es kann vorgesehen sein, dass der sprungartige Übergang in azimutaler Lage um die optische Hauptachse an der Stelle ausgebildet ist, an welcher ein flacher Hauptmeridian des torisch brechenden Oberflächenprofils ausgebildet ist. Sind das torisch brechende Oberflächenprofil und die Helixwindung als Oberflächenstruktur auf einer gemeinsamen Seite des optischen Teils ausgebildet, so ist quasi der Übergang überlagert zu dem flachen Hauptmeridian ausgebildet. Sind die Helixwindung und das torisch brechende Oberflächenprofil an unterschiedlichen Seiten des optischen Teils ausgebildet, so sind sie quasi gegenüberliegend mit dem flachen Hauptmeridian und dem Übergang ausgebildet, so dass bei einer Projektion der beiden Seiten des optischen Teils in eine gemeinsame Ebene dann der sprungartige Übergang und der flache Hauptmeridian wiederum übereinander gelagert ausgebildet sind.

Besonders bevorzugt ist es, wenn der sprungartige Übergang in azimutaler Lage um die optische Hauptachse zu einem Hauptmeridian des torisch brechenden Oberflächenprofils versetzt ausgebildet ist. Dadurch kann die Dicke der Augenlinse nochmals reduziert werden und somit die Kleinschnitttauglichkeit erhöht werden. Insbesondere ist es vorteilhaft, wenn der sprungartige Übergang in azimutaler Lage um die optische Hauptachse in einem azimutalen Winkel zwischen 20° und 70°, insbesondere zwischen40° und 50°, oder zwischen 200° und 250°, insbesondere zwischen 220° und 230°, zu einem flachen Hauptmeridian des torisch brechenden Oberflächenprofils ausgebildet ist. Vorzugsweise ist der Versatz zwischen (1/4 $\pi$) +/- (1/8 $\pi$) oder (5/4 $\pi$) +/- (1/8 $\pi$).

Insbesondere bezieht sich dieser azimutale Winkelversatz auf eine azimutale Mitte des sich azimutal über eine endliche Breite ausbildenden Übergangs, insbesondere eine Mitte bei einer Projektion des Übergangs in eine Ebne senkrecht zur optischen Hauptachse.

Insbesondere ist die Lage der Helixwindung so gebildet, dass ein Windungsanfang in im Uhrzeigersinn um die optische Hauptachse der Augenlinse umlaufend vor dem Windungsende liegt. Die Helixwindung verläuft daher insbesondere ausgehend von dem Windungsanfang gegen den Uhrzeigersinn zum Windungsende.

Ein azimutaler Versatz des Übergangs der Helixwindung und somit auch ein Versatz eines an den Übergang direkt angrenzenden Windungsanfang und einem direkt an den Übergang angrenzenden Windungsende, gegenüber einem Hauptmeridian des torisch brechenden Oberflächenprofils ist im Uhrzeigersinn um die optische Hauptachse der Augenlinse bemessen.

Dadurch wird die Fertigung erleichtert und die Oberflächenform kann durch Fertigungswerkzeuge sehr genau erzeugt werden. Dadurch wird die gewünschte Abbildungseigenschaft der Augenlinse bestmöglich auch in der Praxis erreicht. Insbesondere führt diese Ausführung zu geringen Beschleunigungswerten bei spanabhebenden Fertigungsverfahren. Insbesondere wird die Dicke der Linse minimiert und die Kleinschnitttauglichkeit erhöht.

Vorzugsweise ist vorgesehen, dass sich eine durch die Helixwindung bewirkte azimutale Brechkraft um die optische Hauptachse zwischen einem Windungsanfang und einem Windungsende nicht linear verändert. Durch eine derartige Ausgestaltung kann gerade das Intervall der Schärfentiefe individuell eingestellt und auch an dem Übergang so gestaltet werden, dass die optische Abbildungseigenschaft verbessert ist.

Insbesondere ist vorgesehen, dass der Verlauf der durch die Helixwindung bewirkten azimutalen Brechkraft in einem ersten azimutalen Winkelintervall, welches mit dem Windungsanfang beginnt, und einem zweiten azimutalen Winkelintervall, welches mit dem Windungsende endet, flacher ist, als in einem zwischen den beiden Winkelintervallen liegenden dritten Winkelintervall. Ausgehend von dem Windungsanfang wird der Verlauf der durch die Windung bewirkten azimutalen Brechkraft innerhalb eines Winkelintervalls von dem Windungsanfang bis zu 120°, vorzugsweise bis zu 100°, mit einer geringeren Brechkraftwertveränderung ausgebildet, als in dem dritten Winkelintervall. Selbiges gilt für das zweite Winkelintervall.

Durch eine derartige Ausgestaltung ist insbesondere auch eine Gewichtung der Brechkräfte abhängig von der Größe des Winkelintervalls, über welche sie anliegen, ausgebildet.

Es kann beispielsweise jedoch auch ein vollständig linearer Verlauf der durch die Helixwindung bewirkten azimutalen Brechkraft über einen vollständigen Umlauf um die Hauptachse ausgebildet sein.

Insbesondere ist vorgesehen, dass der Verlauf der azimutalen Brechkraft, die durch die Helixwirkung bewirkt wird, in einer Vertikalachse und somit zur Brechkraftachse in einem Brechkraft-Azimutwinkeldiagramm spiegelsymmetrisch ist. Insbesondere ergibt sich dadurch eine symmetrische Gewichtungsfunktion der Brechkräfte. Vorzugsweise weisen diejenigen Brechkräfte eine höhere Gewichtung auf, die über einen größeren Azimutwinkelbereich und somit ein größeres Winkelintervall anliegen.

Insbesondere ist vorgesehen, dass sich eine Azimut-abhängige und somit eine in Umlaufrichtung um die optische Hauptachse betrachtete Gesamtbrechkraft der Augenlinse gemäß nachstehender Formel 2 ergibt:

$$F_{ges}(\Phi, \rho) = F_{Sphäre}(\rho) + F_{Cylinder}(\Phi, \rho) + F_{HOA}(\Phi, \rho) + F_{EDoF}(\Phi, \rho) \qquad (2)$$

mit

| | |
|---|---|
| $\Phi$ = | Azimutwinkel |
| $\rho$ = | radiale Position auf dem optischen Teil |
| $F_{ges}$ = | azimutabhängige Gesamtbrechkraft |
| $F_{Spähre}$ = | sphärische Brechkraft |
| $F_{Cylinder}$ = | azimutabhängiger zylindrischer Brechkraftanteil |
| $F_{HOA=}$ = | azimutabhängiger Brechkraftanteil zur Korrektur von Aberration höherer Ordnung (bspw. Koma, Fünfblattfehler etc.) |
| $F_{EDoF}$ = | azimutabhängiger Brechkraftanteil zur Beeinflussung der Schärfentiefe |

Die in der Formel 2 genannten Brechkräfte können entsprechend den bekannten optischen Prinzipien einzeln oder in Kombination refraktiv oder diffraktiv erzeugt werden. Der Azimutabhängige Brechkraftanteil zur Beeinflussung der Schärfentiefe und somit der durch die Helixwindung zur Gesamtbrechkraft der Augenlinse beigetragene Brechkraftanteil beziehungsweise diese azimutale Brechkraft ist ein Brechkraftgradient, welcher in Abhängigkeit von der gewünschten Gewichtung der Brechkräfte auch frei gewählt werden kann.

Die Erreichung der Kleinschnitttauglichkeit wird auch durch die Reduktion des Linsenvolumens mittels Geometrie-abhängiger sägezahnartiger Stufung beziehungsweise Fresnellierung der Augenlinse erreicht. Es wird also ein in radialer Richtung gestuftes Profil zumindest der Vorderseite oder zumindest der Rückseite durchgeführt. Insbesondere wird diese Fresnellierung ohne Kopplung an eine in der Formel 2 genannte Teilbrechkraft der Gesamtbrechkraft durchgeführt, sondern in verallgemeinerter Weise entsprechend geometrisch-optischer Randbedingungen in einer Transformation der Oberflächentopografie vorgenommen. Dabei ist bei der Fresnellierung zu berücksichtigen, dass der mit der zunehmenden Radialkoordinate anwachsende Defokus durch die Verschiebung der Krümmungsmittelpunkte in den jeweiligen Fresnellzonen und somit ringförmigen Zonen um eine optische Hauptachse, durch eine Anpassung der Brechkräfte in den jeweiligen ringförmigen Zonen kompensiert ist. Durch die Entkopplung der Fresnellierung von den einzelnen Teilbrechkräften der Gesamtbrechkraft ist erreicht, eine einhüllende Topografie der Augenlinse beziehungsweise des optischen Teils in vorteilhafter Weise hinsichtlich Symmetrie und Injektionseigenschaften in ein Auge zu verbessern. Durch eine geeignete azimutale Variation der Stufentiefe einer Fresnellzone kann eine vorteilhafte Rotationssymmetrie der einhüllenden Linsenkontur erzeugt werden. Derartige Geometrien erleichtern die Handhabbarkeit sowie die Injizierbarkeit der Linse und erlauben somit in Besonderem eine Verbesserung der Kleinschnitttauglichkeit.

Mit einer Fresnellierung ist bevorzugt eine sägezahnartige radiale Oberflächenkontur zu verstehen, bei der ein Phasenhub zwischen benachbarten Stufen und somit auch Zonen größer $n\lambda$, mit $n$ größer oder gleich 2, insbesondere größer oder gleich 4, ist.

Bei der Implantation torischer Intraokularlinsen in das Auge hängt das refraktive Ergebnis neben der korrekten Wahl der sphärischen und zylindrischen Brechkraft auch von der Genauigkeit der Rotationsorientierung zwischen der torischen Korrekturlinse und der torischen Hornhaut ab. Bei einer korrekten Wahl der sphärischen und zylindrischen Brechkraftkomponenten ist ein astigmatischer Restfehler bei Abwesenheit eines Achsfehlers gleich Null. Eine Rotation aus der Idealposition zwischen der Achse der Korrekturlinse und der zylindrischen Hornhaut bezeichnet den Achsfehler. Mit zunehmendem Achsfehler steigt der Wert des Restzylinders an. Bei einem Achsfehler von beispielsweise 30° erreicht der astigmatische Restfehler genau den Wert des zu korrigierenden Zylinderanteils. Das heißt, die Korrektur ist praktisch wirkungslos hinsichtlich der Amplitude und führt zudem zu einer Rotation der resultierenden Zylinderachse. Wird die Korrekturlinse um 90° verdreht eingesetzt, dann addieren sich die Korrekturwerte mit den zu korrigierenden Werten und der zylindrische Restfehler verdoppelt sich.

Zur Gestaltung des durch die Helixwindung bewirkten azimutalen Brechkraftanteils an der Gesamtbrechkraft kann insbesondere jede Oberflächenform dieser Helixwindung ausgebildet werden, welche im Winkelbereich von 0 bis $2\pi$ monoton anwächst oder monoton fällt. Durch die Anstiegscharakteristik und die damit im Winkelintervall verbundene Verteilung in einem bestimmten Brechkraftintervall kann die Intensitätsverteilung im Fokus eingestellt werden.

So kann somit diese Intensitätsverteilung im Fokus im Wesentlichen symmetrisch bezüglich einer Zielbrechkraft und somit einem niedrigsten Brechkraftwert einer azimutalen Brechkraftverteilung, insbesondere einer Helixwindung, verteilt ausgebildet sein oder zugunsten einer Nahdominanz oder einer Ferndominanz verschoben sein.

Es ist vorgesehen, dass zumindest eine Seite des optischen Teils ein in radialer Richtung gestuftes Oberflächenprofil aufweist. Im Querschnitt und somit in einer Schnittebene, die die optische Hauptachse aufweist oder parallel dazu verläuft, ist durch dieses radial gestufte Profil auch eine Art Sägezahnkontur gebildet.

Bei einer Ausgestaltung der Augenlinse, die ein radial gestuftes Oberflächenprofil aufweist, kann vorgesehen sein, dass die Stufen durch um die optische Hauptachse umlaufende ringförmige Zonen gebildet sind, die radial aneinander angrenzen und in Richtung der Hauptachse versetzt zueinander ausgebildet sind.

Es kann insbesondere vorgesehen sein, dass die optisch wirkenden Oberflächen der ringförmigen Zonen der Augenlinse alle gleich groß sind.

In einer vorteilhaften Ausführung ist vorgesehen, dass eine Stufenhöhe einer Stufe einer ringförmigen Zone in azimutaler Richtung und somit in Umlaufrichtung um die optische Hauptachse konstant ist. Das heißt, die Stufenhöhe variiert nicht mit dem Azimutwinkel. Bei einer derartigen Ausgestaltung folgt somit die Fresnellierung der Oberflächenkontur der Seite, auf der die Fresnellierung ausgebildet ist.

Es ist vorgesehen, dass sich eine Stufenhöhe einer Stufe einer ringförmigen Zone in azimutaler Richtung und somit in Umlaufrichtung um die optische Hauptachse verändert. Es ist vorgesehen, dass eine Stufenhöhe in azimutaler Lage eines steileren Hauptmeridians des torisch brechenden Oberflächenprofils größer ist, als eine Stufenhöhe in azimutaler Lage eines flacheren Hauptmeridians des torisch brechenden Oberflächenprofils.

Es kann vorgesehen sein, dass an einem flachen Hauptmeridian des torisch brechenden Oberflächenprofils die Stufenhöhe gleich Null ist, und sie dann bis zu einem steilen Hauptmeridian ansteigt, insbesondere konstant ansteigt.

Es kann auch vorgesehen sein, dass an einem Windungsanfang und somit einem Rand des Übergangs der Helixwindung keine Stufung der Oberflächenkontur und somit keine gestufte radiale Oberflächenform ausgebildet ist, am Windungsende und somit an einem zweiten Rand des Übergangs jedoch eine sägezahnartige Stufung und somit eine Fresnellierung ausgebildet ist.

Ebenso kann in einer weiteren Ausführung eine radiale sägezahnartige Stufung sowohl am Windungsanfang als auch am Windungsende der Helixwindung ausgebildet ist.

Es kann auch eine radiale sägezahnartige Stufung an einem flachen Hauptmeridian des torisch brechenden Oberflächenprofils ausgebildet sein.

Bezüglich einer torischen Linse beziehungsweise eines torisch brechenden Oberflächenprofils ist eine Ausgestaltung gemeint, bei der in zwei senkrecht zueinander stehenden Richtungen zwei unterschiedliche Brechkräfte vorliegen und somit auch in diese senkrecht zueinander stehenden Richtungen (Hauptmeridiane) unterschiedliche Krümmungen, insbesondere unterschiedliche Scheitelkrümmungen, ausgebildet sind.

Vorzugsweise ist vorgesehen, dass die Stufenhöhe so angepasst ist, dass die Linsentopografie und somit die Oberflächengestaltung an eine vorgegebene gewünschte Zielfunktion, die die Oberfläche beschreibt, angepasst ist. Diese Zielfunktion kann sich konstant oder variabel gegenüber dem Azimutwinkel ausbilden.

In radialer Richtung sind die Fresnellsegmente und somit die ringförmigen Zonen vorzugsweise so angeordnet, dass ihre Oberflächen konstant sind beziehungsweise zumindest im Wesentlichen gleich groß sind. Bei anderen Ausführungen kann dies jedoch variieren und somit die Flächengrößen der optischen Oberflächen der jeweiligen ringförmigen Zonen unterschiedlich sein.

Durch eine Fresnellierung ist ein Versatz der ringförmigen Linsenelemente beziehungsweise der Zonen entlang der optischen Hauptachse ausgebildet. Um eine Scheitelbrechkraft der Augenlinse in den ringförmigen Zonen konstant zu halten, ist vorzugsweise eine individuelle Ausgestaltung der Scheitelkrümmungen der einzelnen Zonen insbesondere an den Stufenspitzen beziehungsweise Stufenscheiteln der Stufen ausgebildet.

Darüber hinaus können die optischen Abbildungseigenschaften in den jeweiligen ringförmigen Zonen durch eine Variierung der Asphärisierung abhängig vom Azimutwinkel erfolgen.

Bei einer Ausgestaltung der Augenlinse mit variabler Stufenhöhe der Fresnelzonen lässt sich die sagittale Höhe der Oberfläche bzw. einer strukturierten Seite des optischen Teils vorzugsweise durch nachfolgende Formel 3 bestimmen.

$$s(r, r_0, Q) = \frac{r^2}{r_0 \left(1 + \sqrt{1 - (1 + Q)\frac{r^2}{r_0^2}}\right)} \tag{3}$$

r = radiale Position auf Linsenoberfläche

$r_0$ = zentraler Krümmungsradius ($r_0 \rightarrow r_{target}$ = Krümmungsradius der Zielfunktion (einhüllende Oberfläche))

Q = konische Konstante

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind.

Die in den Unterlagen angegebenen konkreten Werte von Parametern und Angaben zu Verhältnissen von Parametern bzw. Parameterwerten zur Definition von Ausführungsbeispielen der Augenlinse sind auch im Rahmen von Abweichungen, beispielsweise aufgrund von Messfehlern, Systemfehlern, DIN-Toleranzen etc. als vom Rahmen der Erfindung mit umfasst anzusehen, wodurch auch Erläuterungen, die sich auf im Wesentlichen entsprechende Werte und Angaben beziehen darunter zu verstehen sind.

Kurze Beschreibung der Zeichnungen

Ausführungsbeispiele der Erfindung werden nachfolgend anhand schematischer Zeichnungen näher erläutert. Es zeigen:

Fig. 1a      eine perspektivische Darstellung eines ersten Ausführungsbeispiels einer erfindungsgemäßen Augenlinse;

Fig. 1b      eine perspektivische Darstellung eines weiteren Ausführungsbeispiels einer erfindungsgemäßen Augenlinse;

Fig. 2      eine perspektivische Darstellung eines Ausführungsbeispiels einer optischen Oberfläche eines optischen Teils eines Ausführungsbeispiels einer erfindungsgemäßen Augenlinse, bei welcher die optische Oberfläche keine Fresnellierung aufweist;

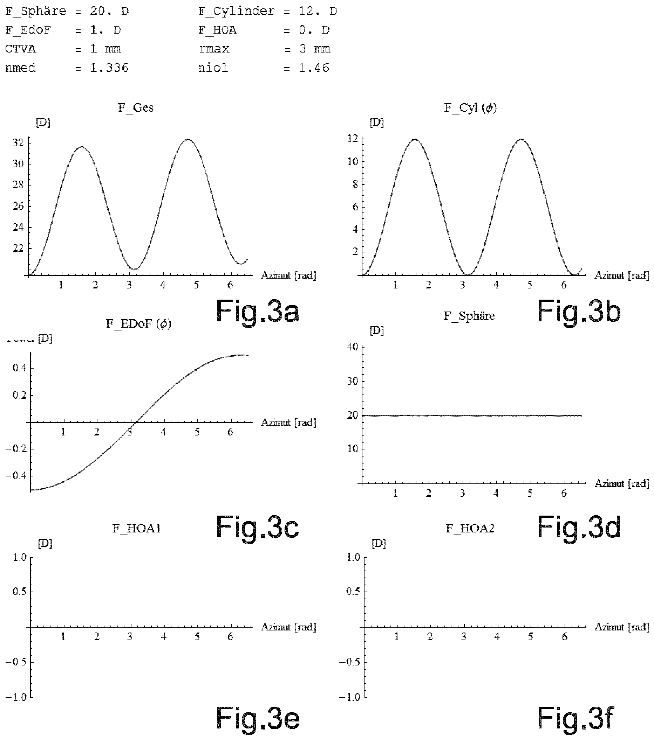

Fig. 3a-3k      eine Mehrzahl von Diagrammen, bei denen die Brechkraft, der Scheitelradius, die Scheitelkrümmung, die konische Konstante und die sagittale Höhe in Abhängigkeit von dem Azimutwinkel und einem Satz von ersten Parameterwerten der sphärischen Brechkraft, der Zylinderbrechkraft, der azimutalen Brechkraft der Helixwindung, der azimutalen Brechkraft zur Korrektur von Aberrationen höherer Ordnung, der Mittendicke der Augenlinse, des maximalen Radius des optischen Teils der Augenlinse, dem Brechungsindex des Materials der Augenlinse und dem Brechungsindex des Immersionsmediums, in dem sich die Augenlinse befindet, angegeben sind;

Fig. 4a-4k      eine Darstellung von Diagrammen, wie sie in Fig. 3a bis 3f gezeigt sind, wobei der Parameterwert der azimutalen Brechkraft der Helixwindung im Vergleich zum Ausführungsbeispiel in Fig. 3a bis 3k verdoppelt ist;

Fig. 5a-5k    eine Darstellung der Diagramme, wie sie in Fig. 3a bis 3k und 4a bis 4k gezeigt sind und der azimutale Brechkraftanteil der Helixwindung gegenüber der Ausführung in Fig. 3a bis 3k verdreifacht ist;

Fig. 6    eine Tabelle mit einer Mehrzahl von Ausführungsbeispielen mit konkreten Parameterwerten für eine Augenlinse, wie sie mit ihrer Oberflächengestaltung eines optischen Teils in Fig. 2 beispielhaft dargestellt ist;

Fig. 7    eine perspektivische Darstellung eines weiteren Ausführungsbeispiels einer optischen Oberfläche eines optischen Teils eines Ausführungsbeispiels einer erfindungsgemäßen Augenlinse, bei welcher die optische Oberfläche in radialer Richtung gestuft ausgebildet ist und somit eine Fresnellierung aufweist;

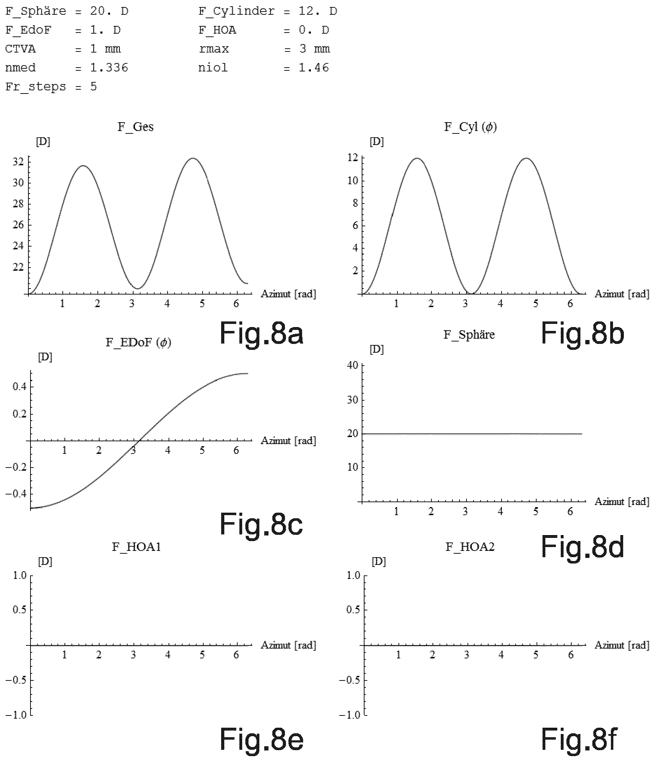

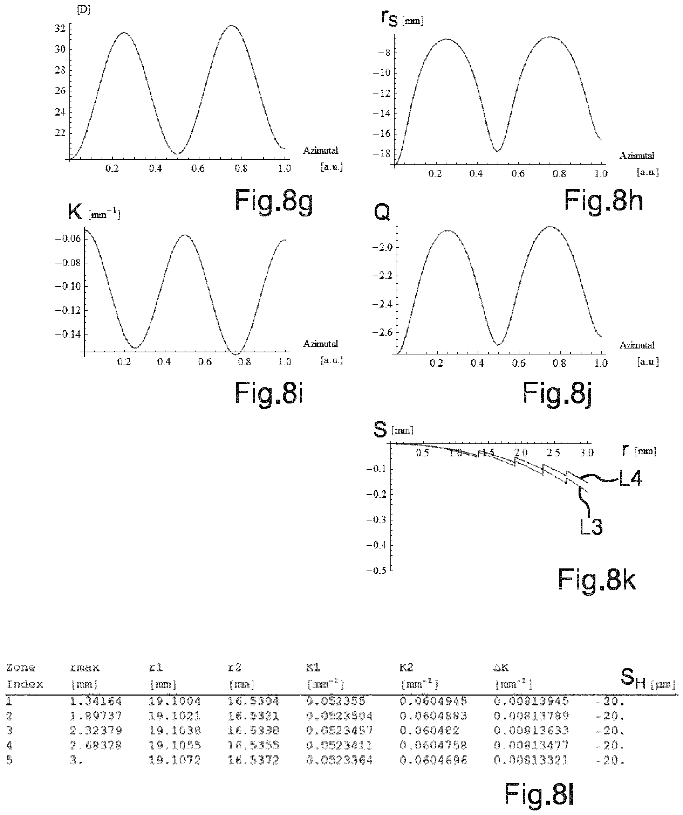

Fig. 8a-8k    eine Mehrzahl von Diagrammen, bei denen die Brechkraft, der Scheitelradius, die Scheitelkrümmung, die konische Konstante und die sagittale Höhe in Abhängigkeit von dem Azimutwinkel und einem Satz von ersten Parameterwerten der sphärischen Brechkraft, der Zylinderbrechkraft, der azimutalen Brechkraft der Helixwindung, der azimutalen Brechkraft zur Korrektur von Aberrationen höherer Ordnung, der Mittendicke der Augenlinse, des maximalen Radius des optischen Teils der Augenlinse, dem Brechungsindex des Materials der Augenlinse und dem Brechungsindex des Immersionsmediums, in dem sich die Augenlinse befindet, angegeben ist, wobei auf der Oberfläche fünf ringförmige Zonen ausgebildet sind, die in radialer Richtung gestuft zueinander ausgebildet sind;

Fig. 8l    eine Tabelle, in welcher konkrete Parameterwerte für die fünf ringförmigen Zonen des Ausführungsbeispiels in Fig. 8a bis 8k angegeben sind;

Fig. 9a-9k    eine Darstellung der Diagramme analog zu Fig. 8a bis 8k mit den zugrunde gelegten Parameterwerten, wobei hier der azimutale Brechkraftanteil der Helixwindung gegenüber der Ausführung in Fig. 8a bis 8k verdoppelt ist;

Fig. 9l    eine Tabelle, in der konkrete Parameterwerte für die ringförmigen Zonen des Ausführungsbeispiels gemäß Fig. 9a bis 9k angegeben sind;

Fig. 10a-10k    eine Darstellung von Diagrammen, wie sie in Fig. 8a bis 8k und 9a bis 9k auf Basis der zugrunde gelegten Parameterwerte dargestellt sind, wobei im Vergleich zu Fig. 8a bis 8k der azimuale Brechkraftanteil der Helixwindung gegenüber der Ausführung in Fig. 8a bis 8k verdreifacht ist;

Fig. 10l    eine Tabelle, in der beispielhafte Parameterwerte für die fünf ringförmigen Zonen des Ausführungsbeispiels in Fig. 10a bis 10k dargestellt sind;

Fig. 11    eine perspektivische Darstellung eines Ausführungsbeispiels einer optischen Oberfläche eines optischen Teils eines weiteren Ausführungsbeispiels einer erfindungsgemäßen Augenlinse, bei der im Vergleich zu den Ausführungen in Fig. 7 bis 10l eine Stufenhöhe einer ringförmigen Zone in azimutaler Richtung variiert, wohingegen sie in Fig. 7 bis Fig. 10l diesbezüglich konstant ist;

Fig. 12a-12k    eine Darstellung von Diagrammen analog zu den Ausführungen in Fig. 8a bis 8k auf Basis der zugrunde gelegten Parameterwerte, wobei die Anzahl der ringförmigen Zonen erhöht ist und die Stufenhöhe variiert;

Fig. 12l    eine Tabelle, in welcher Parameterwerte für die die ringförmigen Zonen beschreibenden Parameter angegeben sind;

Fig. 13a-13k    die Darstellung der Diagramme analog zu Fig. 12a bis 12k, wohingegen die zugrunde gelegten Parameterwerte in der azimutalen Brechkraftdifferenz der Helixwindung unterschiedlich sind und dies in Fig. 13a bis 13k gegenüber der Ausführung in Fig. 12a bis 12k verdoppelt ist;

Fig. 13l    eine Tabelle, in der Parameterwerte für die die ringförmigen Zonen beschreibenden Parameter angegeben sind;

Fig. 14a-14k      eine Darstellung der Diagramme analog zu Fig. 12a bis 12k, wobei hier der grundlegende Parameter der azimutalen Brechkraftdifferenz durch die Helixwindung gegenüber der Ausführung in Fig. 12a bis 12k verdreifacht ist;

Fig. 14l      eine Tabelle, in der Parameterwerte der die ringförmigen Zonen der Ausführung in Fig. 14a bis 14k beschreibenden Parameter angegeben sind;

Fig. 15a -15k      eine Darstellung der Diagramme analog zu Fig. 12a bis 12k, wobei hier der grundlegende Parameter der azimutalen Brechkraftdifferenz durch die Helixwindung gegenüber der Ausführung in Fig. 12a bis 12k verdreifacht ist und eine radiale Stufung auf dem flacheren Radius und somit dem Windungsanfang der Helixwindung ausgebildet ist;

Fig. 15l      eine Tabelle, in der Parameterwerte der die Fresnellierung charakterisierenden ringförmigen Zonen der Ausführung in Fig. 15a bis 15k beschreibenden Parameter angegeben sind;

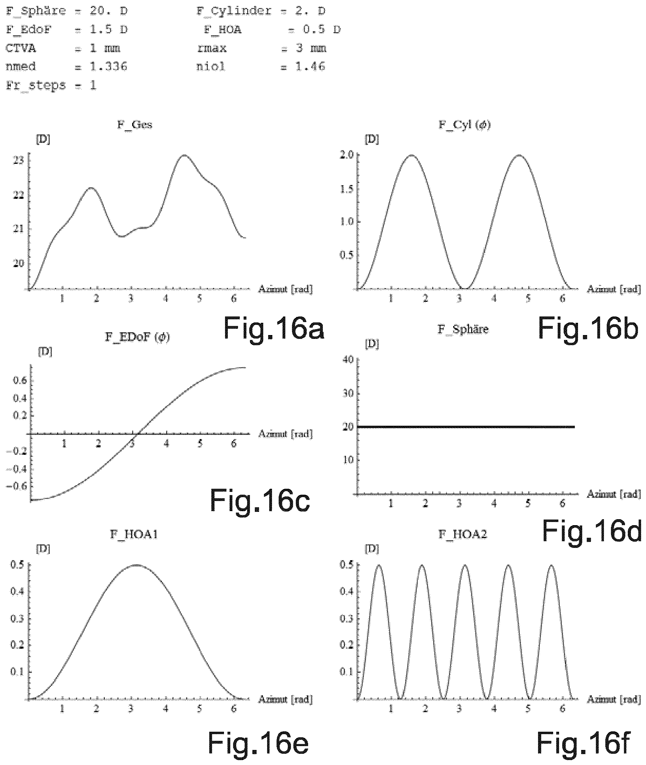

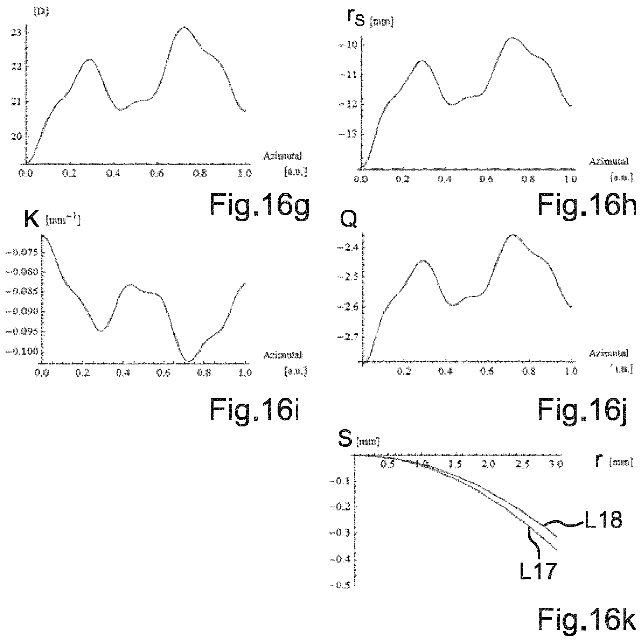

Fig. 16a - 16k      eine Darstellung von Diagrammen in denen die gesamte Brechkraft sowie dazu beitragende Brechkraftanteile und der Scheitelradius, die Scheitelkrümmung, die konische Konstante und die sagittale Höhe in Abhängigkeit von dem Azimutwinkel bzw. dem Radius für einen Satz von ersten Parameterwerten der sphärischen Brechkraft, der Zylinderbrechkraft, der azimutalen Brechkraft der Helixwindung, der azimutalen Brechkraft zur Korrektur von Aberrationen höherer Ordnung, der Mittendicke der Augenlinse, des maximalen Radius des optischen Teils der Augenlinse, dem Brechungsindex des Materials der Augenlinse und dem Brechungsindex des Immersionsmediums, in dem sich die Augenlinse befindet, angegeben sind;

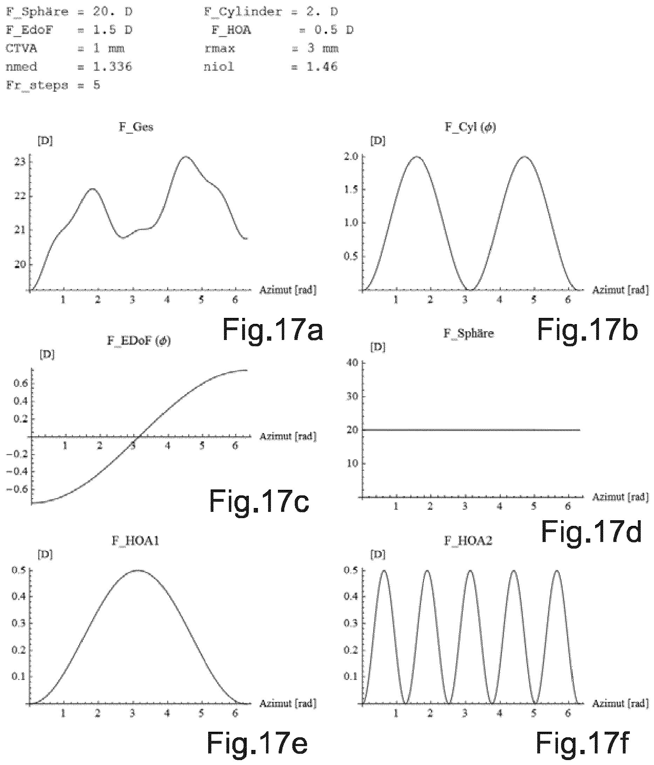

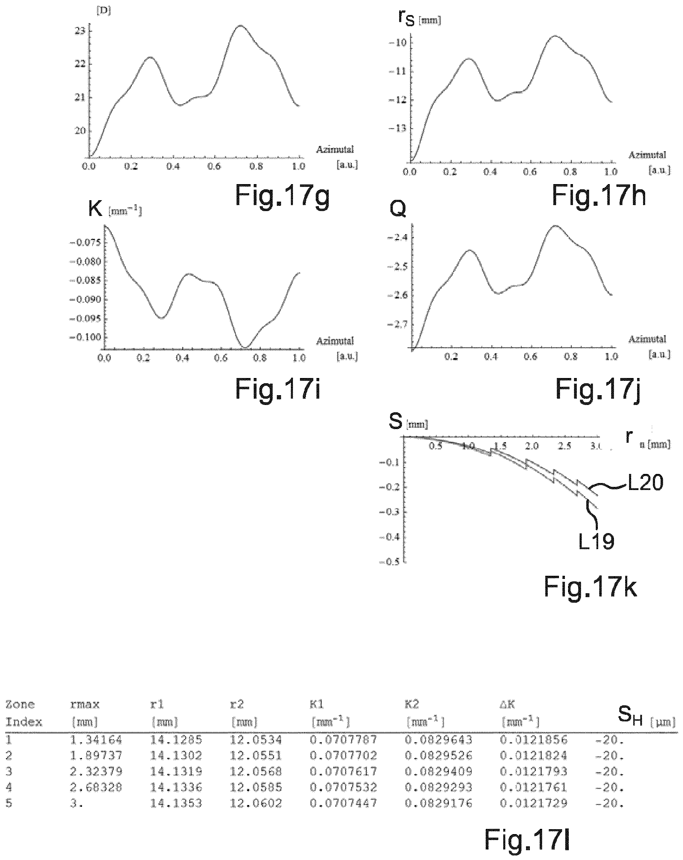

Fig. 17a - 17k      eine Darstellung von Diagrammen in denen die gesamte Brechkraft sowie dazu beitragende Brechkraftanteile und der Scheitelradius, die Scheitelkrümmung, die konische Konstante und die sagittale Höhe in Abhängigkeit von dem Azimutwinkel bzw. dem Radius für einen Satz von ersten Parameterwerten der sphärischen Brechkraft, der Zylinderbrechkraft, der azimutalen Brechkraft der Helixwindung, der azimutalen Brechkraft zur Korrektur von Aberrationen höherer Ordnung, der Mittendicke der Augenlinse, des maximalen Radius des optischen Teils der Augenlinse, dem Brechungsindex des Materials der Augenlinse und dem Brechungsindex des Immersionsmediums, in dem sich die Augenlinse befindet, sowie eine Mehrzahl von radialen Stufen einer Fresnellierung angegeben sind;

Fig. 17l      eine Tabelle, in der Parameterwerte der die radiale Stufung bildenden ringförmigen Zonen der Ausführung in Fig. 17a bis 17k beschreibenden Parameter angegeben sind;

Fig. 18      ein Diagramm, bei dem eine Höhe des Übergangs der Helixwindung abhängig von der azimutalen Lage des Übergangs im Vergleich zu einem Hauptmeridian des torisch brechenden Oberflächenprofils dargestellt ist;

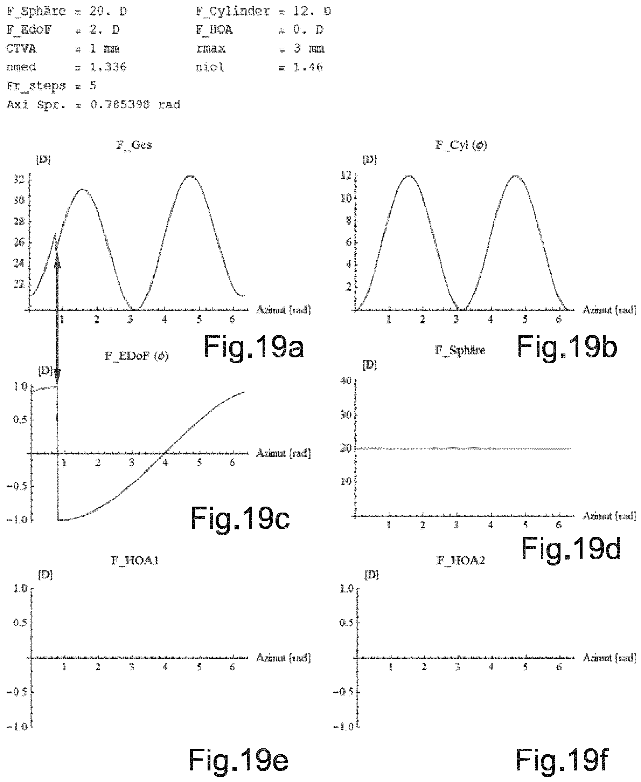

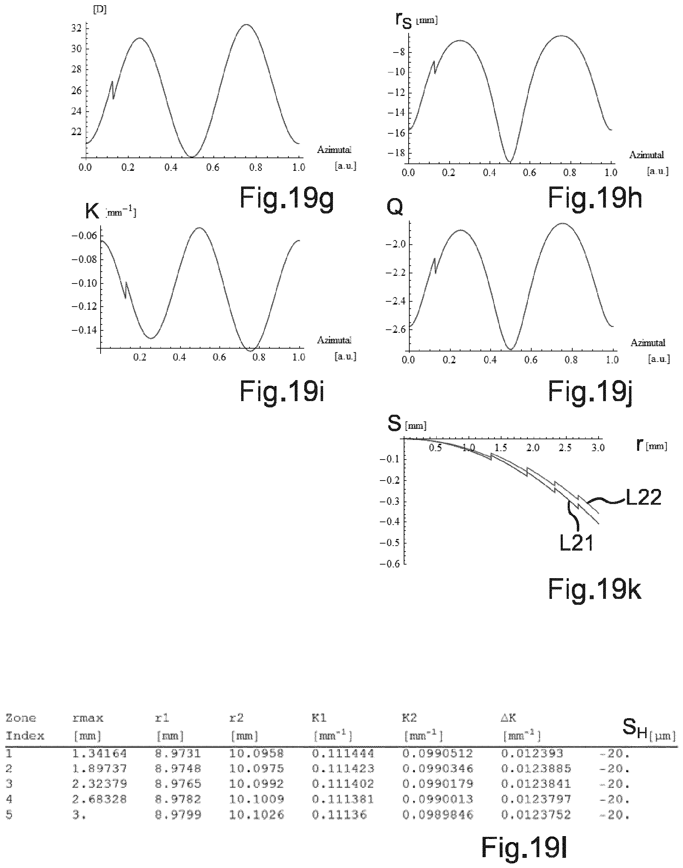

Fig. 19a - 19k      eine Darstellung von Diagrammen in denen die gesamte Brechkraft sowie dazu beitragende Brechkraftanteile und der Scheitelradius, die Scheitelkrümmung, die konische Konstante und die sagittale Höhe in Abhängigkeit von dem Azimutwinkel bzw. dem Radius für einen Satz von ersten Parameterwerten der sphärischen Brechkraft, der Zylinderbrechkraft, der azimutalen Brechkraft der Helixwindung, der azimutalen Brechkraft zur Korrektur von Aberrationen höherer Ordnung, der Mittendicke der Augenlinse, des maximalen Radius des optischen Teils der Augenlinse, dem Brechungsindex des Materials der Augenlinse und dem Brechungsindex des Immersionsmediums, in dem sich die Augenlinse befindet, sowie einer Mehrzahl von radialen Stufen angegeben ist, und ein azimutaler Versatz zwischen dem Übergang der Helixwindung und einem Hauptmeridian des tprisch brechenden Oberflächenprofils ausgebildet ist, gezeigt sind;

Fig. 19l      eine Tabelle, in der Parameterwerte der die radiale Stufung bildenden ringförmigen Zonen der Ausführung in Fig. 19a bis 19k beschreibenden Parameter angegeben sind;

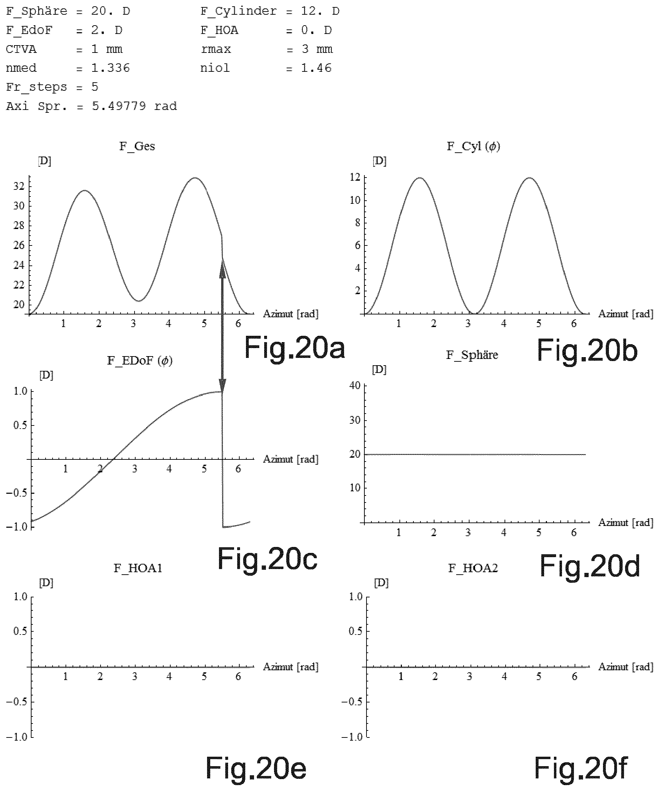

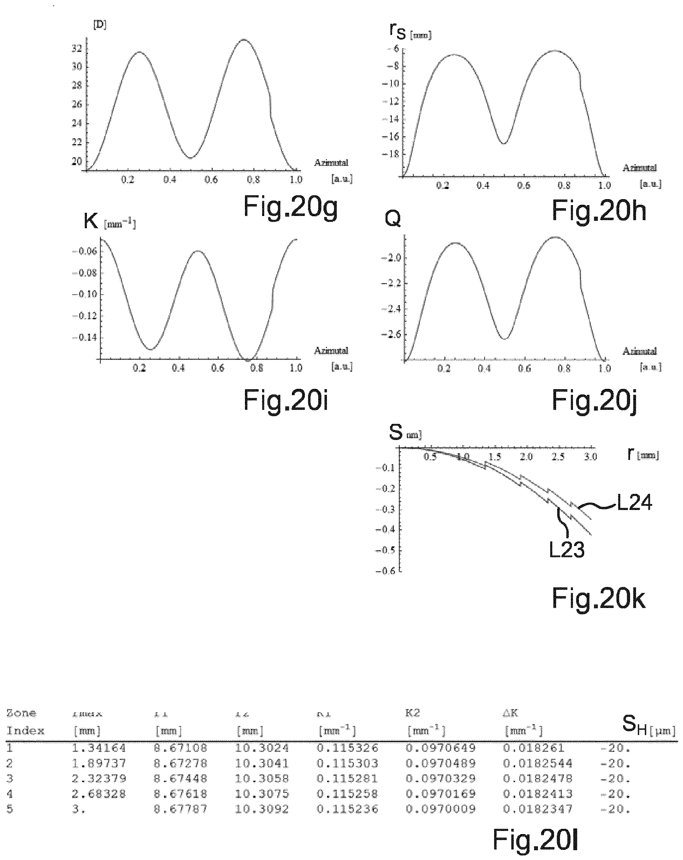

Fig. 20a - 20k      eine Darstellung von Diagrammen in denen die gesamte Brechkraft sowie dazu beitragende Brechkraftanteile und der Scheitelradius, die Scheitelkrümmung, die konische Konstante und die sagittale Höhe in Abhängigkeit von dem Azimutwinkel bzw. dem Radius für einen Satz von ersten Parameterwerten der sphärischen Brechkraft, der Zylinderbrechkraft, der azimutalen Brechkraft der Helixwindung, der azimutalen Brechkraft zur Korrektur von Aberrationen höherer Ordnung, der Mittendicke der Augenlinse, des maximalen Radius des optischen Teils der Augenlinse, dem Brechungsindex des Materials der Augenlinse und dem Brechungsindex des Immersionsmediums, in dem sich die Augenlinse befindet, sowie einer Mehrzahl von radialen Stufen angegeben ist, und ein im Vergleich zu Fig. 19a bis 19l unterschiedlicher azimutaler Versatz zwischen dem Übergang und einem Hauptmeridian ausgebildet ist, gezeigt sind; und

Fig. 20l      eine Tabelle, in der Parameterwerte der die radiale Stufung bildenden ringförmigen Zonen der Ausführung in Fig. 20a bis 20k beschreibenden Parameter angegeben sind.

Bevorzugte Ausführung der Erfindung

In den Figuren werden gleiche oder funktionsgleiche Elemente mit den gleichen Bezugszeichen versehen.

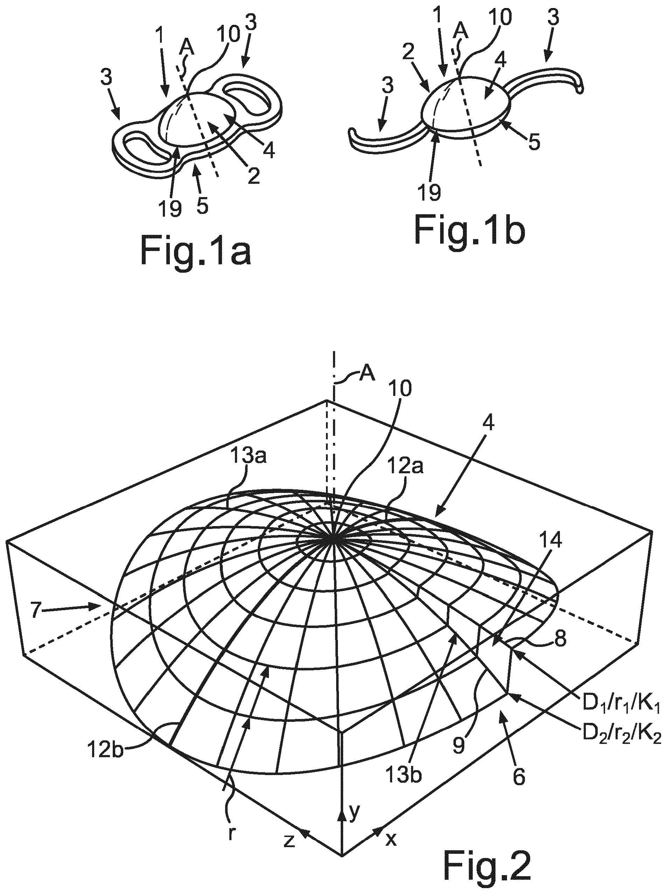

In Fig. 1a ist in einer perspektivischen Darstellung ein erstes Ausführungsbeispiel einer Augenlinse 1 gezeigt, die eine Intraokularlinse ist. Die Augenlinse 1 umfasst einen optischen Teil 2 und daran anschließend eine Haptik 3. Die Augenlinse 1 ist faltbar und über einen Kleinschnitt in ein Auge einführbar. Der optische Teil 2, der wesentlich für die optische Abbildungseigenschaft der Augenlinse 1 ist, umfasst eine optische Hauptachse A. Der optische Teil 2 weist darüber hinaus in Richtung dieser optischen Hauptachse A betrachtet eine erste optische Fläche bzw. Seite 4, die eine Vorderseite sein kann, und gegenüberliegend eine zweite optische Fläche bzw. Seite 5, die eine Rückseite sein kann, auf. Die beispielhafte Vorderseite 4 ist im implantierten Zustand der Augenlinse 1 im Auge der Hornhaut zugewandt, wohingegen die Rückseite dieser Hornhaut abgewandt ist.

In Fig. 1b ist in einer perspektivischen Darstellung ein weiteres Ausführungsbeispiel einer als Intraokularlinse ausgebildeten Augenlinse 1 gezeigt. Sie unterscheidet sich von der Ausführung in Fig. 1a durch die unterschiedliche Haptik 3. Mittels der Haptik 3 ist die Augenlinse 1 im Auge gehalten.

Grundsätzlich können auch anderweitig geformte und ausgestaltete Haptiken 3 vorgesehen sein.

Die Augenlinse 1 mit der optischen Vorderseite 4 und der optischen Rückseite 5 ist so ausgebildet, dass auf zumindest einer dieser beiden Seiten 4, 5 eine Helixwindung als Oberflächenstruktur ausgebildet ist. Durch diese Helixwindung ist eine Brechkraft der Augenlinse 1 in Umlaufrichtung um die Hauptachse A und somit in azimutaler Richtung wertmäßig variierend. Insbesondere ist durch eine derartig vollständig einmalig umlaufende Helixwindung eine azimutale kontinuierliche Brechkraftvariation erreicht, so dass dadurch auch ein spezifischer Schärfentiefenwert erreicht wird. Dieser ist durch die Brechkraftdifferenz, die zwischen einem Windungsanfang und einem Windungsende dieser Helixwindung entsteht und somit den maximalen Unterschied der Brechkräfte dieser Helixwindung darstellt, gegeben.

Darüber hinaus ist vorgesehen, dass zusätzlich zu dieser Helixwindung als Oberflächenstruktur auf zumindest einer Seite 4, 5 ein torisch brechendes Oberflächenprofil ausgebildet ist.

Es kann vorgesehen sein, dass die Helixwindung als Oberflächenstruktur auf der Vorderseite 4 oder der Rückseite 5 aufgebracht ist und das torisch brechende Oberflächenprofil auf der dann gegenüberliegenden Seite 4, 5 ausgebildet ist.

Es kann jedoch auch vorgesehen sein, dass die Helixwindung als Oberflächenstruktur und das torisch brechende Oberflächenprofil auf einer gemeinsamen Seite 4, 5 ausgebildet sind.

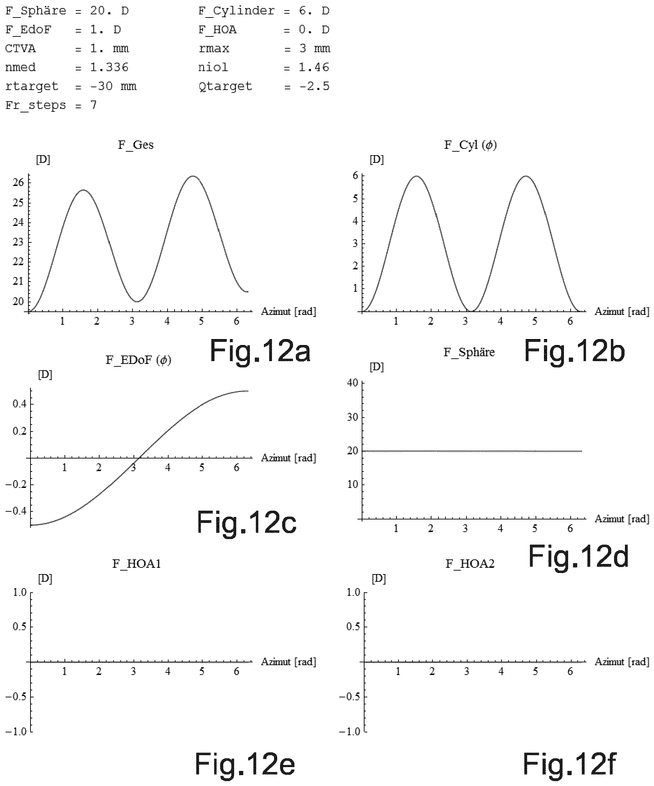

Dazu ist in Fig. 2 beispielhaft die Vorderseite 4 mit ihrer geometrischen Ausgestaltung gezeigt. Bei dieser Ausführung ist die Helixwindung 6 als Oberflächenstruktur und das torisch brechende Oberflächenprofil 7 auf einer gleichen Seite 4, 5 ausgebildet.

Diese Ausgestaltung überlagert somit auf einer Seite, beispielsweise der Vorderseite 4, diese beiden unterschiedlichen Oberflächenformen in Form der Helixwindung 6 und dem torisch brechenden Oberflächenprofil 7. Wie aus der Darstellung in Fig. 2 zu erkennen ist, umfasst die Helixwindung 6 einen Windungsanfang 8, der einen größeren Radius und somit eine kleinere Krümmung aufweist, als ein Windungsende 9. Das Windungsende 9 weist einen kleineren Radius und somit eine größere Krümmung auf. Insbesondere werden diese Krümmungen zumindest in einem Scheitel 10 der Vorderseite 4 betrachtet und verglichen.

Die Helixwindung 6 umfasst darüber hinaus zwischen dem Windungsanfang 8 und dem Windungsende 9 einen sprungartigen Übergang 11, der in azimutaler Richtung und somit in Umlaufrichtung um die Hauptachse A möglichst klein bemessen ist, wobei darüber hinaus darauf zu achten ist, dass die maschinentechnische Herstellung ermöglicht ist.

An diesem Übergang 11 der mit seinen begrenzenden Rändern an den Windungsanfang 8 und das Windungsende 9 direkt anschließt, bildet sich auch durch die unterschiedliche Krümmung des Windungsanfangs 8 und des Windungsendes 9 eine maximale Brechkraftdifferenz aus. Dadurch ist auch der maximale Schärfentiefenwert, der durch die Helixwindung 6 beigetragen wird und gemäß der oben genannten Formel 2 additiv zu den anderen Teilbrechkräften addiert wird und daraus die Gesamtbrechkraft der Augenlinse 1 hervorgeht, gegeben.

Der Windungsanfang 8 charakterisiert sich in dem Zusammenhang durch, insbesondere im Scheitel 10, eine Brechkraft $D1$, einen Scheitelradius $r1$ und eine Scheitelkrümmung $K1$. Demgegenüber charakterisiert sich das Windungsende 9, insbesondere im Scheitel 10, durch eine im Vergleich dazu größere Brechkraft D2, einen kleineren Scheitelradius r2 und eine größere Scheitelkrümmung K2.

Das torisch brechende Oberflächenprofil 7 weist senkrecht zueinander stehende Hauptmeridiane 12 und 13 auf, wobei der steilere und somit mit einer stärkeren Krümmung insbesondere im Scheitel 10 ausgebildete Hauptmeridian 12a, 12b mit einer größeren Brechkraft einhergeht als der flachere Hauptmeridian 13a, 13b.

Wie darüber hinaus zu erkennen ist, ist in Fig. 2 eine Ausgestaltung realisiert, bei der der Übergang 11 in azimutaler Richtung überlagert mit einem flachen Hauptmeridian 13b ist. Es kann jedoch auch ein azimutaler Versatz des Übergangs 11, insbesondere einer azimutalen Mitte des Übergangs 11, zu einem Hauptmeridian, insbesondere dem Hauptmeridian 13b, ausgebildet sein, der vorzugsweise 1/4 $\pi$ +/- (1/8 $\pi$) oder (5/4 $\pi$) +/- (1/8 $\pi$) beträgt.

Bei dem Ausführungsbeispiel ist auch vorgesehen, dass die kleineren Hauptmeridiane 12a und 12b, die in einem 180°-Winkel zueinander versetzt and, unterschiedliche Krümmungen aufweisen, die größer sind als die wiederum unterschiedlich zueinander ausgebildeten Krümmungen der 180° zueinander stehenden Hauptmeridane 13a und 13b.

Bei dem in Fig. 2 gezeigten Ausführungsbeispiel ist die Vorderseite 4 in radialer Richtung r ohne eine Stufung und somit ohne eine Fresnellierung ausgebildet.

Vorzugsweise ist vorgesehen, dass die Seite 4, 5, auf welcher zumindest die Helixwindung 6 ausgebildet ist, einen Scheitel 10 aufweist, und bei allen Ausführungen ein Differenzwert von einer Scheitelkrümmung am Windungsanfang 8 und einer Scheitelkrümmung am Windungsende 9 zwischen 0,001 mm$^{-1}$ und 0,16 mm$^{-1}$ beträgt.

Ebenso für alle Ausführungsbeispiele gültig ist, dass die Seite 4, 5, auf welcher zumindest die Helixwindung 6 ausgebildet ist, einen Scheitel 10 aufweist, und eine Differenz von einer Scheitelkrümmung am Windungsanfang 8 und einer Scheitelkrümmung am Windungsende 9 unabhängig von einer Brechkraft D1 am Windungsanfang 8 ist.

Insbesondere gilt auch für alle Ausführungsbeispiele, dass die Differenz der Scheitelkrümmungen unabhängig von der Brechkraft D1 am Windungsanfang 8 als Konstante gegeben ist.

Vorzugsweise ist die Differenz der Scheitelkrümmungen abhängig von der Differenz der Brechkräfte am Windungsanfang 8 und am Windungsende 9 variiert, insbesondere für eine Differenz der Brechkräfte zwischen 1 Dioptrien und 10 Dioptrien zwischen 0,015 mm$^{-1}$ und 0,085 mm$^{-1}$ variiert.

Insbesondere ist auch vorgesehen, dass sich eine durch die Helixwindung 6 bewirkte azimutale Brechkraft um die optische Hauptachse A zwischen einem Windungsanfang 8 und einem Windungsende 9 nicht-linear verändert. Dazu ist insbesondere vorgesehen, dass der Verlauf der durch die Helixwindung 6 bewirkten azimutalen Brechkraft in einem ersten azimutalen Winkelintervall, welches mit dem Windungsanfang 8 beginnt, und einem zweiten azimutalen Winkelintervall, welches mit dem Windungsende 9 endet, flacher ist, als in einem zwischen den beiden Winkelintervallen liegenden dritten Winkelintervall. Dies ist beispielsweise aus der Darstellung in Fig. 3c eines spezifischen Ausführungsbeispiels zu erkennen. In dem Zusammenhang ist in den Fig. 3a bis 3k eine Darstellung mehrerer Diagramme vorgenommen, in denen Brechkräfte der Augenlinse 1, Scheitelradien $r_S$, Scheitelkrümmungen K, konische Konstanten Q und sagittale Höhen s in Abhängigkeit des Azimuts beziehungsweise des Azimutwinkels und der radialen Position r angegeben sind.

In dem Ausführungsbeispiel gemäß Fig. 3a bis 3k wird für die sphärische Brechkraft $F_{Sphäre}$ ein konstanter Wert von 20 Dioptrien zugrunde gelegt. Beispielhaft wird für die Azimut-abhängige zylindrische Brechkraft $F_{cylinder}$ und somit die durch die torische brechende Oberfläche beziehungsweise das Oberflächenprofil 7 beitragende Brachkraftanteil an der Gesamtbrechkraft der Augenlinse 1 ein Maximalwert von 12 Dioptrien zugrunde gelegt. Der azimutale Verlauf dieser Azimut-abhängigen zylindrischen Brechkraft $F_{cylinder}$ ist im Diagramm gemäß Fig. 3b gezeigt, wobei hier der Azimut in den Einheiten "rad" angegeben ist.

Darüber hinaus ist der azimutale Brechkraftanteil der Helixwindung 6 an der Gesamtbrechkraft $F_{Ges}$ der Augenlinse 1 und somit die azimutale Brechkraftdifferenz, wie sie durch die Differenz am Windungsanfang 8 und am Windungsende 9 der Helixwindung 6 gegeben ist, durch 1 Dioptrien vorgegeben. Darüber hinaus ist der gesamte Verlauf dieser durch die Helixwindung 6 erzeugten azimutalen Brechkraftverteilung in dem Diagramm gemäß Fig. 3c gezeigt. Hier ist auch der nicht-lineare azimutale Verlauf zu erkennen, wobei insbesondere ein spiegelsymmetrischer Verlauf der Kurve in Fig. 3c zu einer vertikalen Symmetrieachse zu erwähnen ist. Azimut-abhängige Brechkräfte zur Korrektur von Aberration höherer Ordnung $F_{HOA}$ (bspw. Koma, Fünfblattfehler etc.) sind im Ausführungsbeispiel mit 0 Dioptrien angegeben.

Mit dem Parameter CTVA ist auch die Mittendicke ct der Augenlinse 1 bezeichnet, die im Ausführungsbeispiel 1 mm beträgt. Mit der Mittendicke wird die Dicke des optischen Teils 2 entlang der optischen Hauptachse A bezeichnet. Darüber hinaus ist der optische Teil 2 mit einem maximalen Radius von 3 mm angegeben. Der Brechungsindex $n_{iol}$ des Polymermaterials des optischen Teils 2 beträgt 1,46. Darüber hinaus ist der Brechungsindex $n_{med}$ angegeben, der für das Kammerwasser im menschlichen Auge charakteristisch ist und den Wert 1,336 beträgt.

Die Diagramme in Fig. 3e und 3f zeigen in dem Zusammenhang den bereits erwähnten Nullanteil der Azimut-abhängigen Brechkräfte zur Korrektur von Aberration höherer Ordnung.

In Fig. 3a ist dann die Summe der Brechkräfte, wie sie in den Fig. 3b bis 3f gezeigt sind, angegeben, wobei hier eine Summation gemäß der Formel 2 zugrunde liegt.

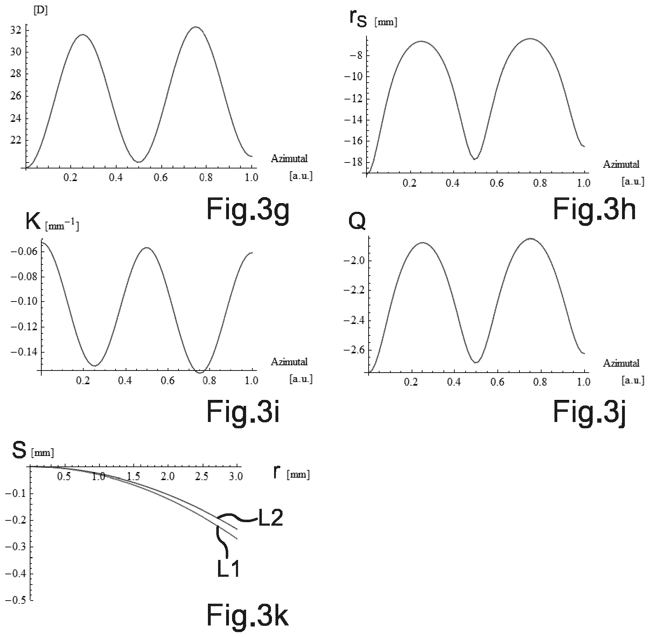

In Fig. 3g ist diese gesamte Brechkraft $F_{Ges}$ der Augenlinse auch in Abhängigkeit des normierten Azimutwinkels gezeigt. Darüber hinaus ist in Fig. 3h der Verlauf des Scheitelradius $r_S$ in Abhängigkeit von diesem normierten Azimutwinkel dargestellt.

Fig. 3i zeigt ein Diagramm, bei dem die Scheitelkrümmung K in Abhängigkeit des Azimutwinkels dargestellt ist.

In Fig. 3j ist die konische Konstante Q in Abhängigkeit des Azimutwinkels in ihrem Verlauf dargestellt.

Ferner ist in Fig. 3k die sagittale Höhe s des optischen Teils 2 in Abhängigkeit vom der radialen Position r gezeigt, wobei hier die Kurve L1 den Verlauf der sagittalen Höhe des Windungsendes 9 und die Kurve L2 den Verlauf der sagittalen Höhe des Windungsanfangs 8 darstellt.

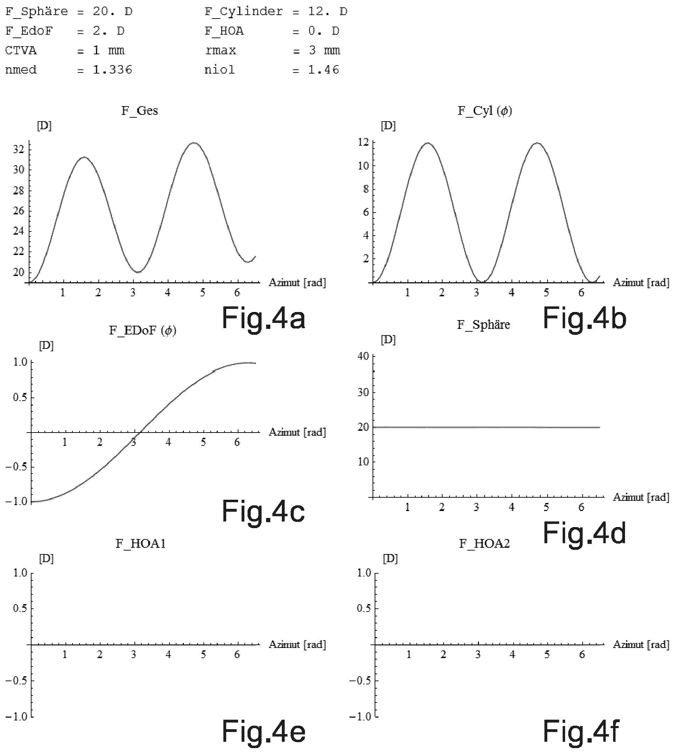

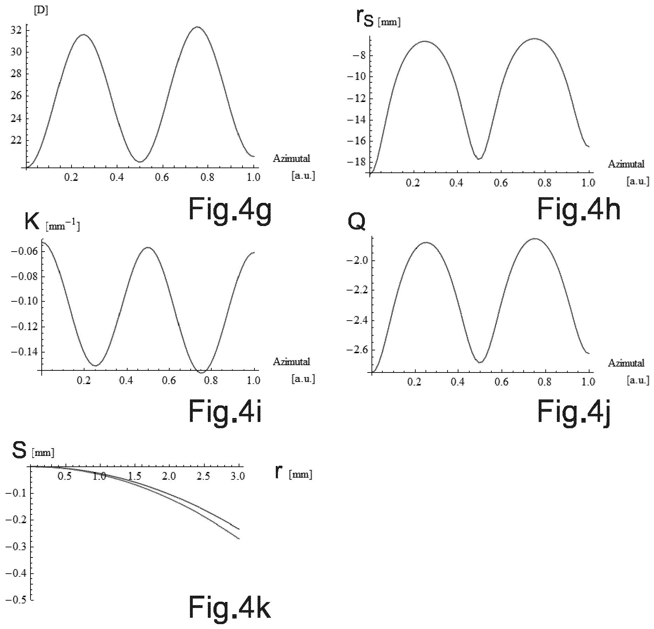

In Fig. 4a bis 4k sind Diagramme analog zu Fig. 3a bis 3k gezeigt. Wie darüber hinaus bezüglich der aufgelisteten Parameterwerte zu den Brechkräften, der Mittendicke, den Brechungsindizes und dem maximalen Radius des optischen Teils 2 zu erkennen ist, unterscheidet sich das Ausführungsbeispiel in Fig. 4a bis 4k gegenüber dem Ausführungsbeispiel in Fig. 3a bis 3k lediglich in der Verdopplung der Brechkraftdifferenz zwischen dem Windungsanfang 8 und dem Windungsende 9, so dass hier $F_{Edof}$ 2 Dioptrien beträgt. Bezüglich der weiteren Erläuterungen zu den Diagrammen und Kurvenverläufen in Fig. 4a bis 4k darf auf die Erläuterungen zu Fig. 3a bis 3k verwiesen werden.

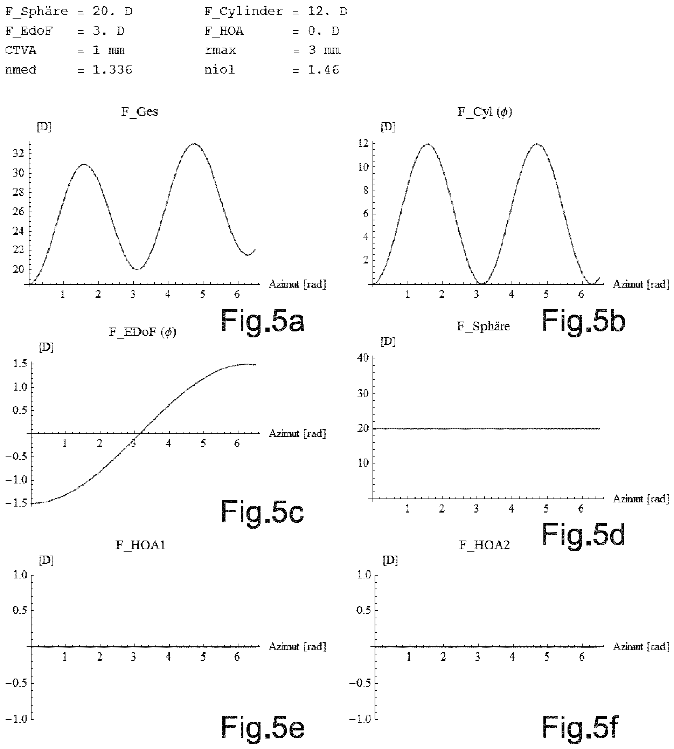

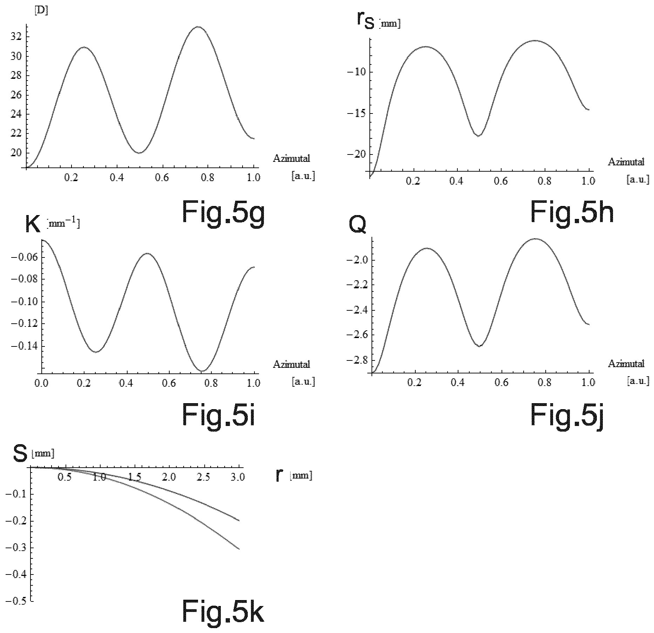

Darüber hinaus ist in den Diagrammen gemäß Fig. 5a bis 5k ein weiteres Ausführungsbeispiel für eine Augenlinse 1 mit einer in radialer Richtung stufenlosen und somit fresnellzonenfreie Ausführung, wie sie in Fig. 2 für eine Oberfläche beziehungsweise eine Vorderseite 4 gezeigt ist, dargestellt. Auch hier darf bezüglich der Erläuterungen zu den Diagrammen auf die Ausführungen zu Fig. 3a bis 3k verwiesen werden. Das Ausführungsbeispiel in Fig. 5a bis 5k unterscheidet sich von dem Ausführungsbeispiel in Fig. 3a bis 3k dahingehend, dass die Brechkraftdifferenz zwischen dem Windungsanfang 8 und dem Windungsende 9 hier nunmehr 3 Dioptrien beträgt und somit gegenüber dem Ausführungsbeispiel in Fig. 3a bis 3k verdreifacht ist.

In den Diagrammen gemäß Fig. 4k und 5k sind die steileren Kurven diejenigen am Windungsende 9 und die flacheren am Windungsanfang 8.

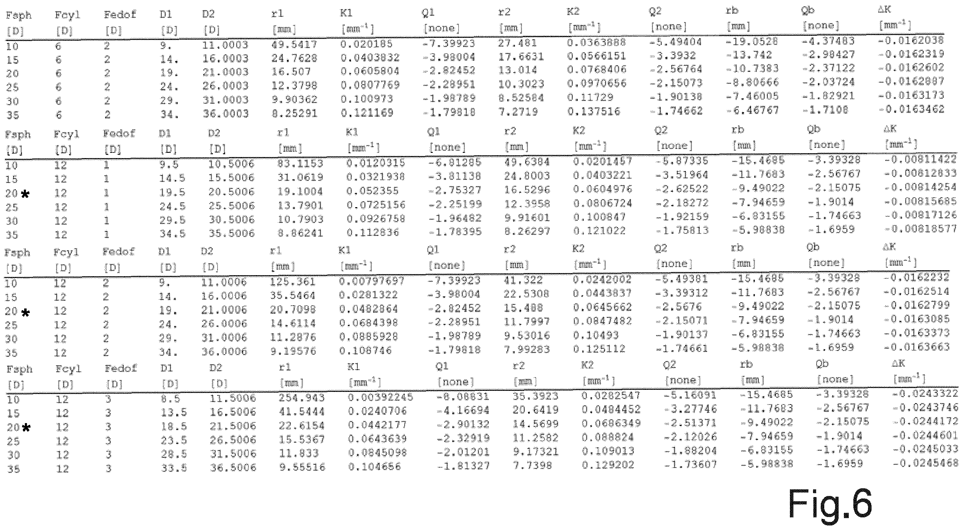

In Fig. 6 ist eine Tabelle einer weiteren Mehrzahl von Ausführungsbeispielen für eine Augenlinse 1 mit einer Gestaltung einer beispielsweise Vorderseite 4 gemäß Fig. 2 gezeigt.

$F_{sph}$ ist hier wiederum die sphärische Brechkraft, $F_{cyl}$ bezeichnet die Azimut-abhängige zylindrische Brechkraft, wie sie durch das torisch brechende Oberflächenprofil 7 beigetragen wird, $F_{edof}$ bezeichnet wiederum die Azimut-abhängige Brechkraft zur Beeinflussung der Schärfentiefe, wie sie durch die Helixwindung 6 als Brechkraftanteil zur Gesamtbrechkraft beigetragen wird. D1 bezeichnet die Brechkraft am Windungsanfang 8, D2 bezeichnet die Brechkraft am Windungsende 9. r1 bezeichnet den Scheitelradius insbesondere am Scheitel 10, und zwar bezogen auf den Windungsanfang 8, wobei K1 die Scheitelkrümmung dazu darstellt. Q1 bezeichnet die konische Konstante vom Windungsanfang 8. Demgegenüber bezeichnen r2 den Scheitelradius am Windungsende 9 insbesondere im Scheitel 10, K2 die Krümmung dazu und somit den inversen Wert zum Scheitelradius r2, wobei Q2 die konische Konstante des Windungsendes 9 bezeichnet.

Darüber hinaus bezeichnet rb den rotationssymmetrischen Radius der im Ausführungsbeispiel dann unstrukturierten Rückseite 5, die dann insbesondere sphärisch ausgebildet ist. Dazu bezeichnet Qb dann die konische Konstante. ΔK bezeichnet die Differenz der Scheitelkrümmungen K1, K2, wie sie beispielhaft und insbesondere durch die Formel 1 bestimmbar ist.

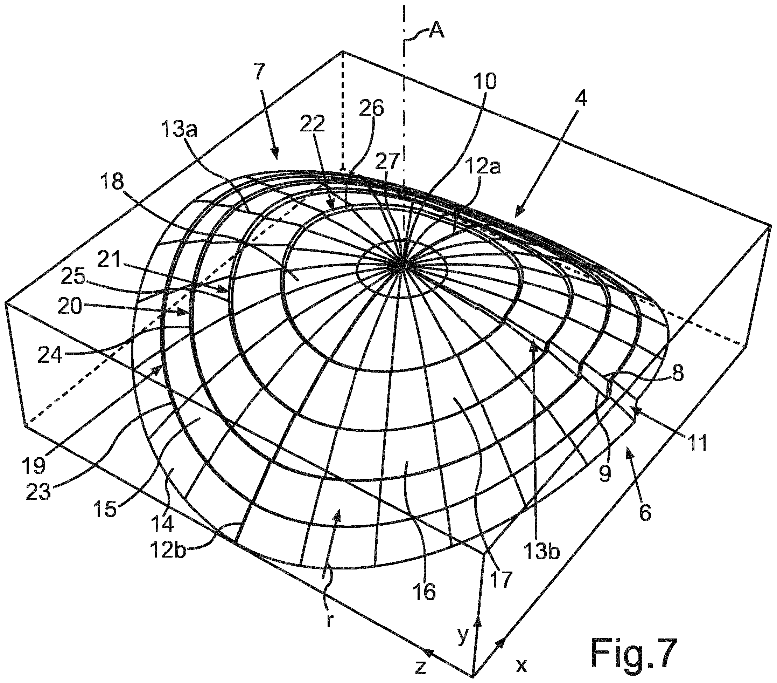

In Fig. 7 ist ein weiteres Ausführungsbeispiel für eine Augenlinse 1 gezeigt, bei der eine Seite, beispielsweise die Vorderseite 4, neben einer Helixwindung 6 und einem torisch brechenden überlagerten Oberflächenprofil 7 in radialer Richtung r auch gestuft ausgebildet ist und dies azimutal zumindest bereichsweise umlaufend um die Achse A ausgebildet ist.

Im gezeigten Ausführungsbeispiel sind für diese gestufte Profilierung und somit Fresnellierung der Vorderseite 4 fünf ringförmige und somit um die Hauptachse A umlaufende Zonen 14, 15, 16, 17 und eine innerste Zone 18 vorgesehen. Die innerste Zone 18 ist in dem Zusammenhang nicht als Ringzone, sondern als kreisartige Fläche zu sehen, wird im weitere jedoch auch als innerste ringförmige Zone erläutert.

Es kann vorzugsweise vorgesehen sein, dass die Flächen der Zonen 14 bis 18 gleich groß sind.

Es ist hier eine Ausführung gezeigt, bei der der Übergang 11, insbesondere dessen azimutale Mitte, azimutal auf gleicher Lage ausgebildet ist als der flache Hauptmeridian 13b. Es kann jedoch auch eine Ausführung realisiert ein, bei welcher ein azimutaler Versatz, insbesondere um (1/4 $\pi$) +/- (1/8 $\pi$) oder (5/4 $\pi$) +/- (1/8 $\pi$) ausgebildet ist.

Es ist bei der gezeigten Ausführung des weiteren eine radiale Stufung sowohl am Windungsanfang 8 als auch am Windungsende 9 realisiert. Die Stufenhöhe einer Zone ist in Fig. 7 über den Azimut konstant und somit gleich hoch.

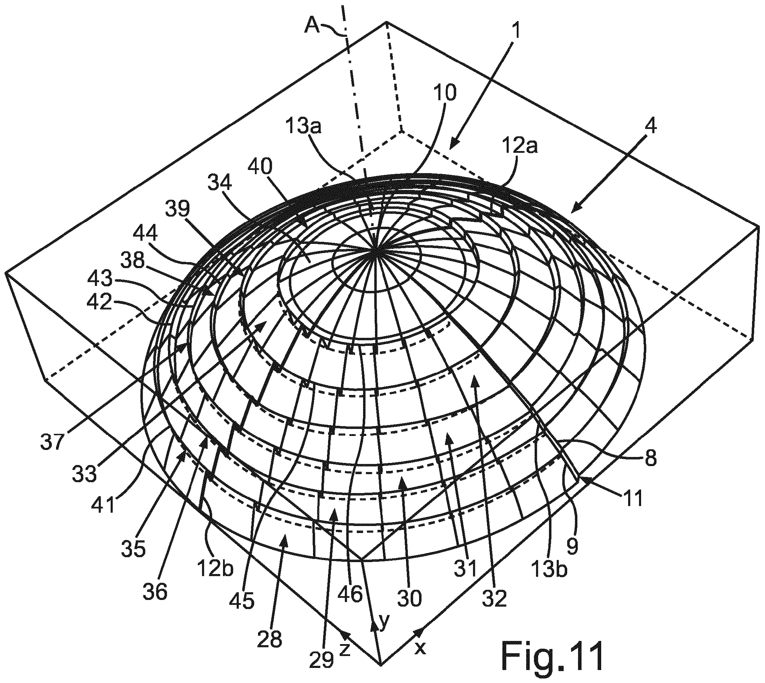

Es werden im nachfolgenden, insbesondere anhand des Beispiels gemäß Fig. 11 und darauf aufbauend bzw. in Abwandlung dazu, auch noch Ausführungen erläutert, die gemäß der Darstellung in Fig. 7 beispielsweise am Windungsanfang 8 keine Stufung aufweise und somit insbesondere der flache Radius des Übergangs 11 stufenlos ist und/oder die Stufenhöhe einer Zone in azimutaler Richtung variiert ist und somit die Stufenhöhe sich in Umlaufrichtung um die Achse A ändert.

Die Ausgestaltung und Anordnung der Zonen 14 bis 18 in Fig. 7 ist darüber hinaus so gewählt, dass an Übergängen zwischen zwei benachbarten und radial aneinander angrenzenden Zonen 14 bis 18 Stufen 19, 20, 21, 22 ausgebildet sind.

Eine Stufenhöhe $S_H$ und somit die Bemessung in Richtung der optischen Hauptachse A beispielsweise der Stufe 22 ist bei dem in Fig. 7 gezeigten Ausführungsbeispiel in Umlaufrichtung um die Achse A konstant, wie bereits erwähnt. Dies bedeutet, dass sie in azimutaler Richtung und somit in Umlaufrichtung um die Achse A an jeder Stelle zwischen dem Windungsanfang 8 und dem Windungsende 9 gleich hoch ist.

Entsprechend gilt dies insbesondere auch für die weiteren Stufen 19 bis 21.

In Fig. 8a bis 8k sind wiederum Diagramme gezeigt, die analog zu den Diagrammen 3a bis 3k, 4a bis 4k und 5a bis 5k zu verstehen sind und ein Ausführungsbeispiel in Zahlen für eine Ausführung einer Seite 4, 5 gemäß der Gestaltung in Fig. 7 darstellt.

Im Ausführungsbeispiel gemäß Fig. 8a bis 8k sind die zugrunde gelegten Parameterwerte für Brechkräfte $F_{sphare}$, $F_{Zylinder}$, $F_{Edof}$, $F_{HOA}$, CTVA, $r_{max}$, $n_{med}$, $n_{iol}$ identisch zu dem Ausführungsbeispiel in Fig. 3a bis 3k. Bei dem Ausführungsbeispiel in Fig. 8a bis 8k werden beispielhaft, wie bereits erwähnt, fünf ringförmige Zonen 14 bis 18 ausgebildet.

Dazu ist gemäß der Darstellung in Fig. 8k eine Kurve L3 gezeigt, die die sagittale Höhe in radialer Richtung am Windungsende 9 zeigt, wohingegen die Kurve L4 die sagittale Höhe in Abhängigkeit vom Radius am Windungsanfang 8 zeigt.

In der Tabelle gemäß Fig. 8l sind beispielhafte Parameterwerte für die fünf ringförmigen Zonen 14 bis 18 dargestellt. Dabei bezeichnet $r_{max}$ jeweils den maximalen Radius der jeweiligen Zone 14 bis 18, r1 den jeweiligen Scheitelradius bei einem jeweiligen Stufenscheitel 23, 24, 25 und 26 sowie 27 der Stufen 19 bis 22 am Windungsanfangs 8. Diese Stufenscheitel 23 bis 27 stellen somit quasi auch insbesondere die Spitzen beziehungsweise oberen Ränder der Stufen 19 bis 22 beziehungsweise des Scheitels 10 dar. Dies jedoch jeweils in dem Windungsanfang 8.

Ebenso beschreibt der Radius r2 die Stufenscheitel 23 bis 27 der Stufen 19 bis 22 beziehungsweise des Scheitels 10 am Windungsende 9. K1 und K2 bezeichnen dann jeweils die Scheitelkrümmungen dazu.

Ferner bezeichnet der Parameter $S_H$ die jeweiligen konstanten Stufenhöhen der Stufen 19 bis 22 in den einzelnen Zonen 14 bis 18.

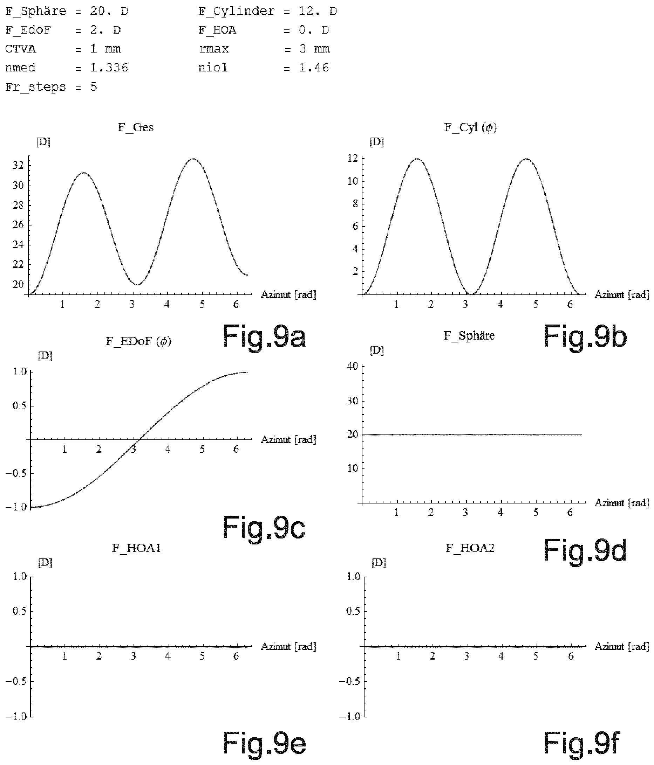

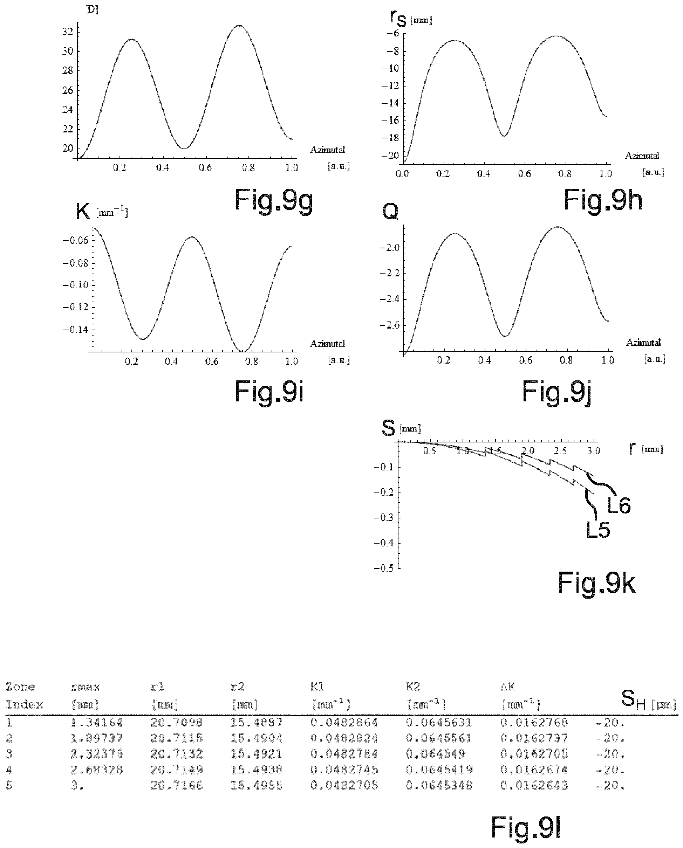

In Fig. 9a bis 9k sind Diagramme analog zu Fig. 8a bis 8k gezeigt. Im Unterschied zum Ausführungsbeispiel in Fig. 8a bis 8k ist lediglich der Parameter der Brechkraftdifferenz der azimutalen Brechkraft der Helixwindung 6 verändert, und zwar verdoppelt, so dass $F_{Edof}$ bei diesem Ausführungsbeispiel 2 Dioptrien beträgt.

In Fig. 9k ist die sagittale Höhe in Abhängigkeit vom Radius r gezeigt und die Konturenverläufe gemäß den Kurven L5 am Windungsende 9 und L6 am Windungsanfang 8 gezeigt.

Darüber hinaus sind in Fig. 9l in einer Tabelle wiederum konkrete Parameterwerte für die Parameter, wie sie bereits zu Fig. 8l erläutert wurden, für das Ausführungsbeispiel gemäß Fig. 9a bis 9k angegeben.

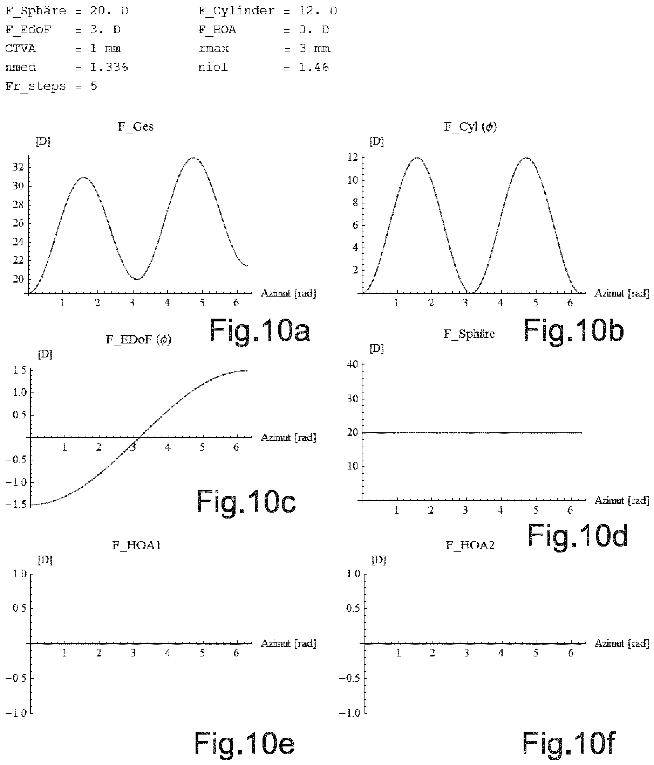

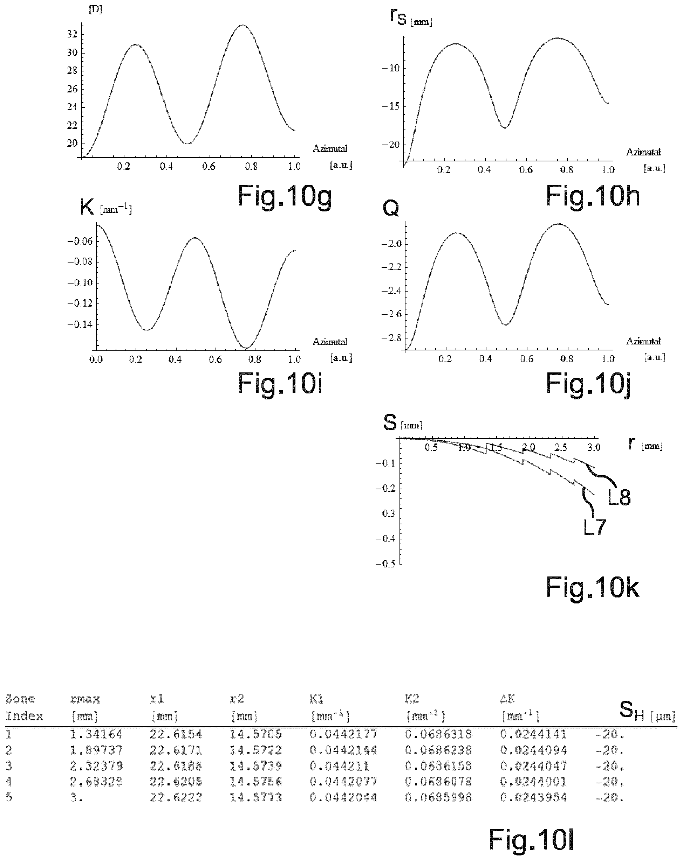

In Fig. 10a bis 10f ist wiederum eine Mehrzahl von Diagrammen analog zu Fig. 8a bis 8k angegeben, wobei hier der Unterschied zu dem Ausführungsbeispiel in Fig. 8a bis 8k darin liegt, dass der Wert von $F_{Edof}$ verdreifacht ist und somit 3 Dioptrien beträgt.

In Fig. 10k ist die sagittale Höhe in Abhängigkeit vom Radius r gezeigt und die Konturenverläufe gemäß den Kurven L7 am Windungsende 9 und L8 am Windungsanfang 8 gezeigt.

Darüber hinaus sind in Fig. 10l in einer Tabelle wiederum beispielhafte Werte für die beispielhafte Anzahl von fünf Zonen 14 bis 18 angegeben.

In Fig. 11 ist in einer weiteren beispielhaften Ausführung eine Augenlinse 1 gezeigt, bei der beispielsweise eine Vorderseite 4 wiederum mit einer Helixwindung 6 und einem überlagerten und torisch brechenden Oberflächenprofil 7 ausgebildet ist. Auch hier ist wiederum ein in radialer Richtung gestuftes Profil überlagert, wie dies auch bereits im Ausführungsbeispiel gemäß Fig. 7 vorgesehen ist. Allerdings ist im Unterschied zur Ausführung gemäß Fig. 7 hier vorgesehen, dass eine Stufenhöhe und somit eine in Richtung der Achse A bemessene Höhe einer Stufe 19 bis 22 in azimutaler Richtung variiert. So ist in diesem Ausführungsbeispiel vorgesehen, dass beispielsweise, hier in der Anzahl auch beispielhaft zu verstehen, sieben ringförmige Zonen 28, 29, 30, 31, 32, 33 und 34 ausgebildet sind.

Auch hier sind bei den aneinander angrenzenden Zonen 28 bis 34 dann jeweils Stufen 35, 36, 37, 38, 39 und 40 ausgebildet.

Insbesondere ist auch hier, wie dies auch bevorzugt in dem Beispiel in Fig. 7 bis Fig. 10l vorgesehen ist, eine Scheitelbrechkraft an jeweiligen Stufenscheiteln 41, 42, 43, 44, 45 und 46 der Stufen 35 bis 40 gleich. Dazu werden die Scheitelkrümmungen an den Stufenscheiteln 41 bis 46 entsprechend geformt und angepasst.

Bei dem Ausführungsbeispiel in Fig. 11 ist darüber hinaus vorgesehen, dass in azimutaler Richtung und somit in Umlaufrichtung die optische Hauptachse A eine Stufenhöhe einer Stufe 35 bis 40 variiert. Insbesondere ist vorgesehen, dass beispielsweise in azimutaler Richtung eine Stufenhöhe in einem steileren Hauptmeridian 12a und/oder 12b größer ist als in einem senkrecht dazu stehenden flacheren Hauptmeridian 13a und/oder 13b. Vorzugsweise verändert sich dieser Stufenhöhenwert kontinuierlich beispielsweise zwischen dem Meridian 12a und 13a, wobei er dann von dem Meridian 13a zum Meridian 12b wieder ansteigt und vom Meridian 12b zum Meridian 13b wieder kleiner wird.

Entsprechendes ist insbesondere auch für die Variation der Stufenhöhe der Stufen 35 bis 40 vorgesehen.

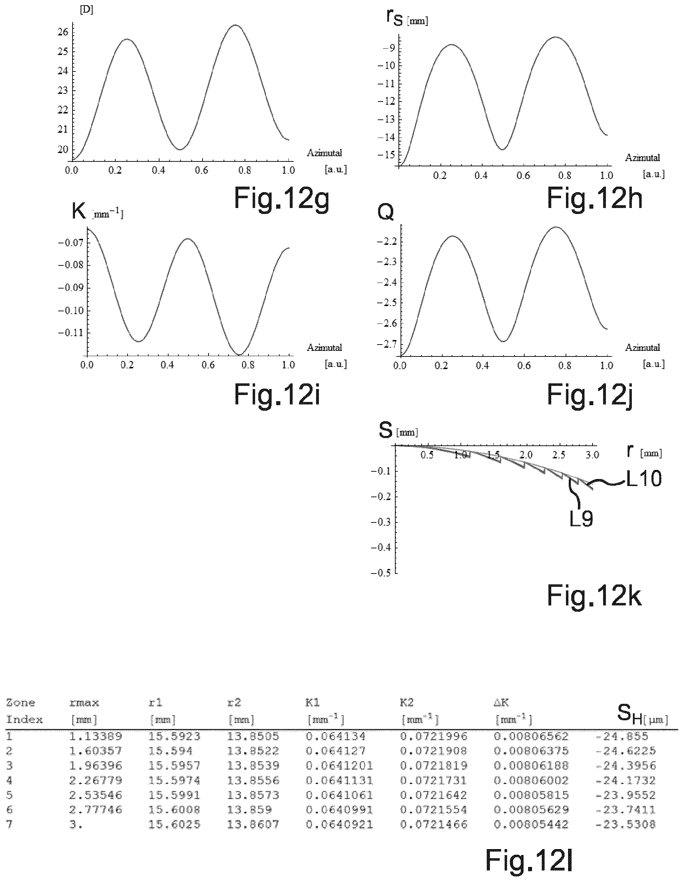

In Fig. 12a bis 12k sind wiederum Diagramme gezeigt, die Parameterwerte analog zu den Ausführungen in Fig. 8a bis 8k zeigen. Auch die zugrunde gelegten Parameterwerte für $F_{Sphäre}$, $F_{zylinder}$, $F_{Edof}$ und $F_{HOA1}$, $F_{HOA2}$ sowie CTVA, $r_{max}$, $n_{med}$, $n_{iol}$ ist identisch zum Ausführungsbeispiel gemäß Fig. 8a bis 8k. Ein Unterschied stellt sich in der Anzahl der Fresnellstufen und somit auch der Anzahl der ringförmigen Zonen dar.

In Fig. 12k ist die sagittale Höhe in Abhängigkeit vom Radius gezeigt, wobei hier die Kurve L9 den Verlauf am Windungsende 9 und die Kurve L10 den Verlauf am Windungsanfang 8 zeigt, der gestuft oder ungestuft sein kann.

In Fig. 12l ist in einer Tabelle wieder die Angabe von konkreten Parameterwerten für die einzelnen Zonen 28 bis 34 gegeben, wie sie bezüglich der Parameterbezeichnungen bereits zu Fig. 8l, 9l und 10l erläutert wurden.

Es ist insbesondere vorgesehen, dass am Windungsanfang 8 keine Stufung ausgebildet ist, am Windungsende 9 jedoch schon. Im gezeigten Beispiel sind somit die Werte für die Stufenhöhe $S_H$ am Windungsende 9, was auch für die nachfolgenden noch erläuterten Ausführungen gilt, und somit auch am Beginn des Übergangs 11 zu verstehen. Die weiteren Werte beziehen sich auf die Stufenhöhe $S_{HHF}$ ("h_Fstep $0\pi$") am flachen Hauptmeridian und auf die Stufenhöhe $S_{HHS}$ ("h_Fstep $\pi/2$") am steilen Hauptmeridian.

Die Stufenhöhen $S_H$ sind maximal so hoch wie die Kurve L10, so dass sie nicht in axialer Richtung über den Windungsanfang 8 und somit auch dem oberen Rand des Übergangs 11 überstehen.

Dies gilt auch insbesondere für alle Ausführungen, so dass Stufungen am Windungsende 9 nicht axial höher sind als Stufungen am Windungsanfang 8 oder ein stufenloser Verlauf der Kontur am Windungsanfang 8.

Es kann auch vorgesehen sein, dass auch am Windungsanfang 8 eine Stufung ausgebildet ist, so dass dann auch dort die Stufenhöhen der Stufen 35 bis 40 größer als Null sind.

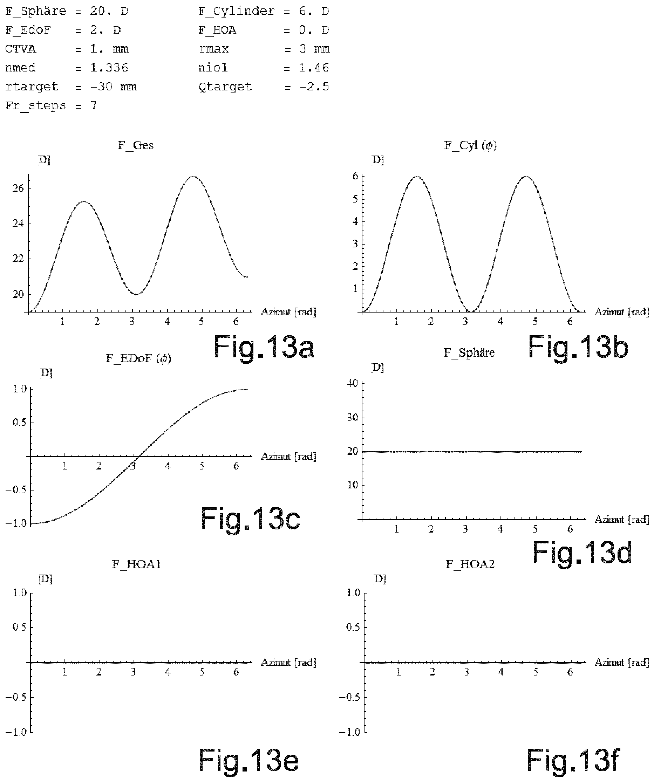

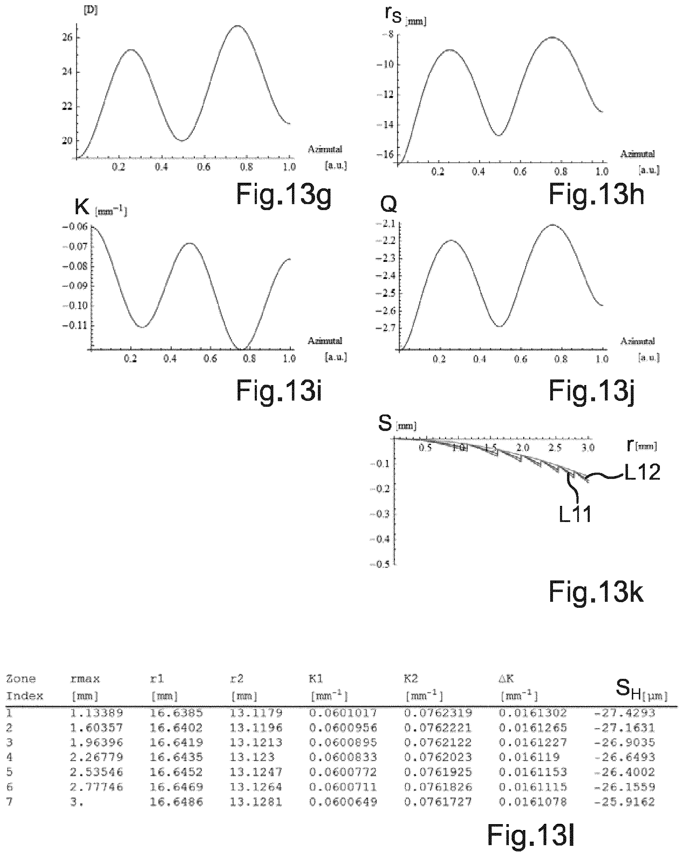

In Fig. 13a bis 13k sind entsprechende Diagramme gezeigt, wobei der Unterschied zum Ausführungsbeispiel in Fig. 12a bis 12k wiederum in dem unterschiedlichen Wert des Parameters $F_{Edof}$ zu finden ist, der hier 2 Dioptrien beträgt. Hier sind durch die Kurven L11 und L12 die sagittalen Höhen in Fig. 13k in Abhängigkeit vom Radius am Windungsende 9 und am Windungsanfang 8 gezeigt. Die Tabelle gemäß Fig. 13l gibt wieder konkrete Parameterwerte für die aufgelisteten Parameter, die die Zonen 28 bis 34 charakterisieren, an.

Bei dieser Ausführung ist bevorzugt eine Stufung sowohl am Windunganfang 8 als auch am Windungsende 9 und somit auch an den Begrenzungsrändern des Übergangs 11 ausgebildet. Die Stufenhöhen einer Stufe und somit einer ringförmigen Zonen können am Windungsanfang 8 und am Windungsende 9 so ausgebildet sein, dass die Stufenspitzen auf gleichem axialen Niveau liegen, das heißt gleiche sagittale Lage aufweisen. Das kann für alle Zonen und somit alle Stufen der Fall sein, wie dies in Fig. 13k ersichtlich ist.

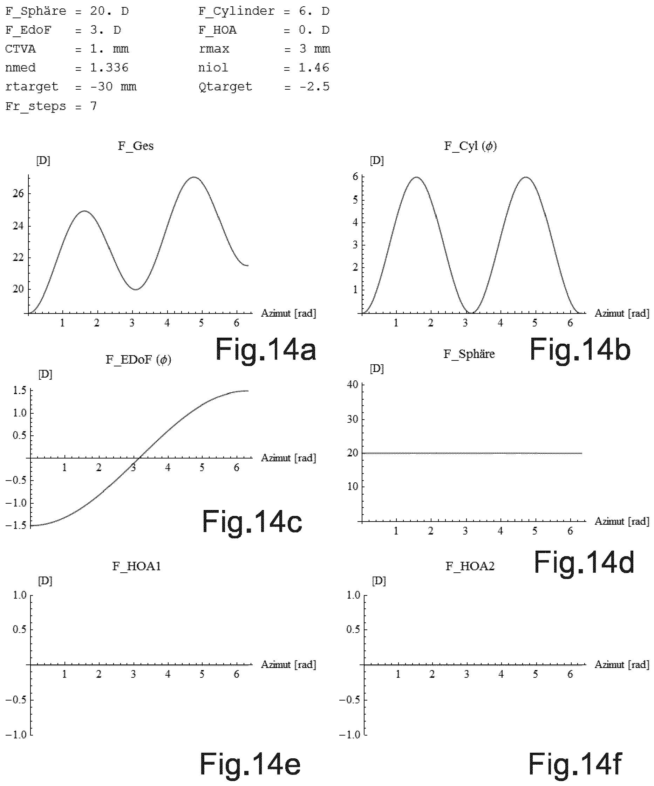

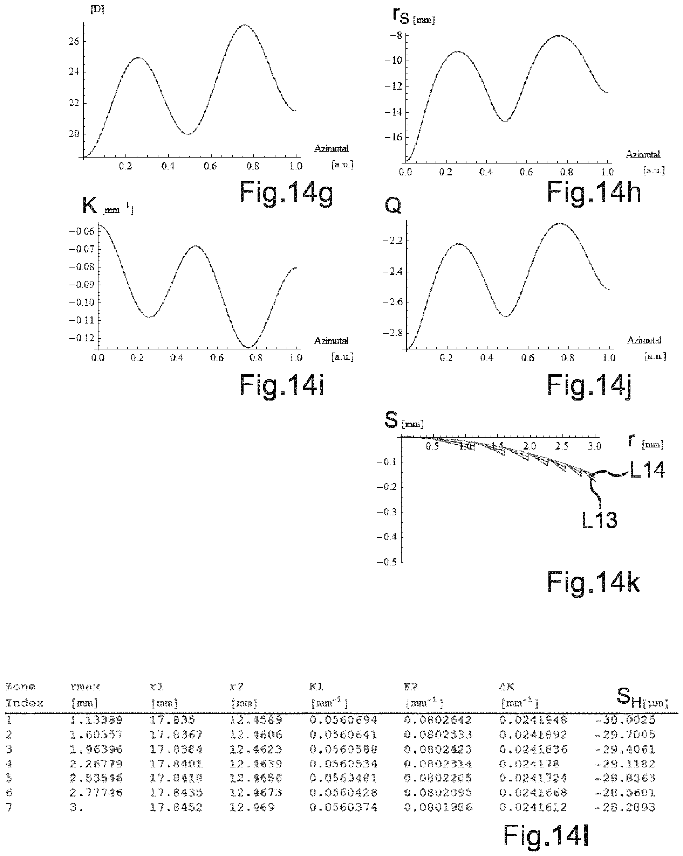

In Fig. 14a bis 14k sind wiederum entsprechende Diagramme wie in Fig. 12a bis 12k dargestellt, wobei sich ein Unterschied in dem Parameterwert $F_{Edof}$ ergibt, der hier 3 Dioptrien ist.

In Fig. 14k ist wiederum die sagittale Höhe in Abhängigkeit vom Radius gezeigt, wobei hier die Kurve L13 am Windungsende 9 und die Kurve L14 am Windungsanfang 8 dargestellt ist.

Auch bei dieser Ausführung ist bevorzugt eine Stufung sowohl am Windunganfang 8 als auch am Windungsende 9 und somit auch an den Begrenzungsrändern des Übergangs 11 ausgebildet. Die Stufenhöhen einer Stufe und somit einer ringförmigen Zonen können am Windungsanfang 8 und am Windungsende 9 so ausgebildet sein, dass die Stufenspitzen auf gleichem axialen Niveau liegen, das heißt gleiche sagittale Lage aufweisen. Das kann für alle Zonen und somit alle Stufen der Fall sein, wie dies in Fig. 13k ersichtlich ist.

In Fig. 14l ist wiederum eine Tabelle mit charakteristischen Parameterwerten für die Zonen 28 bis 34 für das Ausführungsbeispiel gemäß Fig. 14a bis 14k dargestellt.

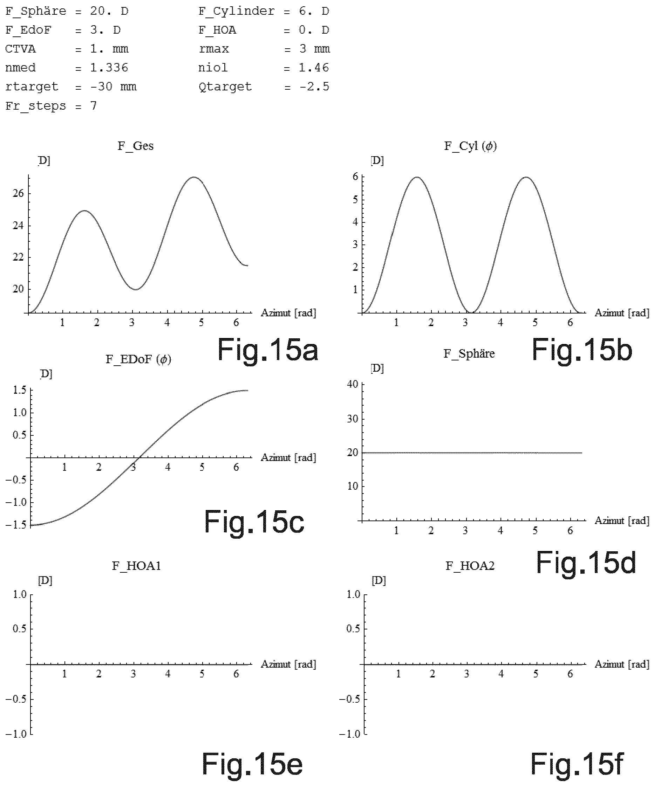

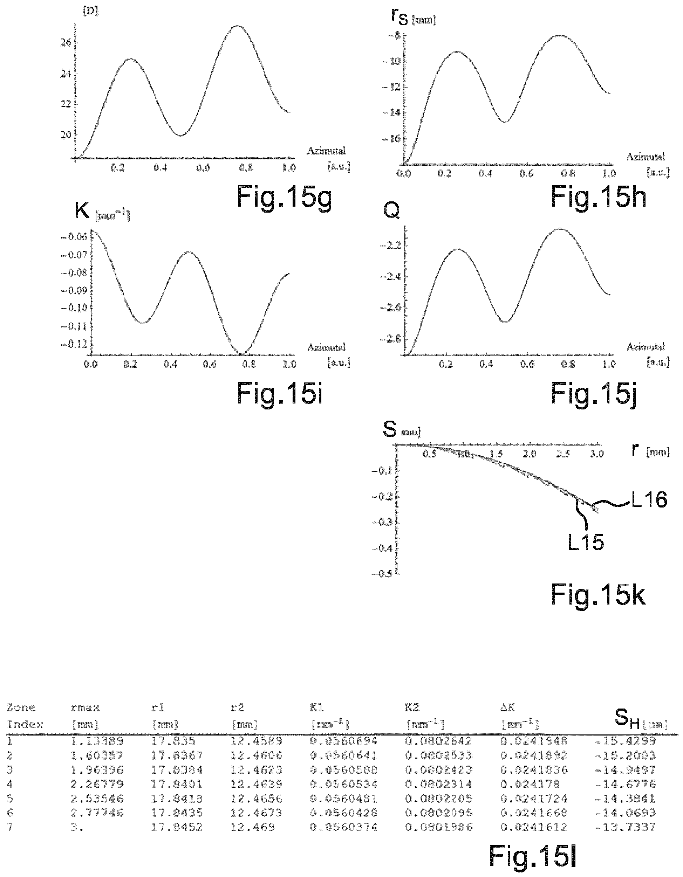

In Fig. 15a bis 15k ist ein Ausführungsbeispiel dargelegt, bei welchem die Ausführung in Fig. 14a bis 14k zu Grunde gelegt ist, jedoch auch keine Fresnellierung und somit die Ausbildung einer sägezahnartigen radialen Stufung am Windungsanfang 8 nicht ausgebildet ist, insbesondere auch der Übergang 11 und der flache Hauptmeridian 13b azimutal die gleiche Lage aufweisen. Dadurch ergeben sich unterschiedliche Werte für die Stufenhöhen $S_H$, wie dies aus den Tabellen in Fig. 14l und Fig. 15l erkennbar ist.

In Fig. 16a bis 16k ist ein weiteres Ausführungsbeispiel gezeigt, bei welchem auch eine azimutale Brechkraft bezüglich einer Korrektur von Aberrationen höhere Ordnung ungleich Null ist.

In Fig. 17a bis 17k ist ein weiteres Ausführungsbeispiel gezeigt, bei welchem auch eine azimutale Brechkraft bezüglich einer Korrektur von Aberrationen höhere Ordnung ungleich Null ist und bei welchem im Unterschied zum Beispiel in Fig. 16a bis 16k eine Fresnellierung ausgebildet ist. Es ist hier im Unterschied zu Fig. 16k, in welcher die Kurven L17 und L18 die sagittalen Höhen am Windungsende 9 und am Windungsanfang 8 zeigen, in den Kurven L19 und L20 in Fig. 17k der sägezahnartige Verlauf zu erkennen.

In Fig. 17l ist wiederum eine Tabelle mit charakteristischen Parameterwerten für die fünf Zonen für das Ausführungsbeispiel gemäß Fig. 17a bis 17k dargestellt.

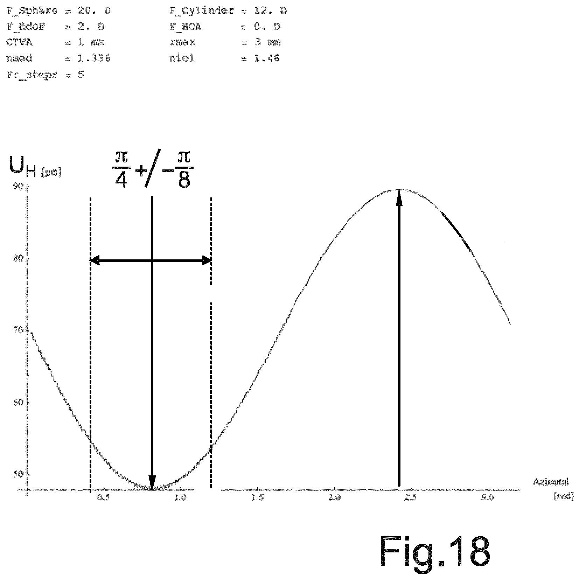

In Fig. 18 ist ein Diagramm gezeigt, bei welchem eine Höhe $U_H$ des Übergangs 11 in Abhängigkeit seiner azimutalen Lage für die genannten Parameterwerte für $F_{Sphäre}$, $F_{zylinder}$, $F_{Edof}$ und $F_{HOA}$, sowie CTVA, $r_{max}$, $n_{med}$, $n_{iol}$ und der Stufen bzw. Zonenanzahl der Fresnelstufen bzw. Fresnelzonen aufgetragen ist. Ein erster bevorzugte Bereich

zwischen (1/4 $\pi$) +/- (1/8 $\pi$) ist dargestellt. Ein weiterer bevorzugter Bereich wäre zwischen (5/4 $\pi$) +/- (1/8 $\pi$), der in dem Diagramm nicht mehre dargestellt ist. Ein minimaler Höhenunterschied ergibt sich gerade für 1/4 $\pi$. Dadurch werden, im Vergleich zu anderen Ausführungen, die entsprechenden Abbildungseigenschaften der Augenlinse 1 mit der Helixwindung 6 und dem torisch brechenden Oberflächenprofil 7 erreicht hat und die axial minimale Dicke des optischen Teils 2 im Vergleich zu anderen Ausführungen erreicht. Gleiches gilt für die Versatzlage bei 5/4 $\pi$.

Zumindest im Wesentlichen, und somit insbesondere im Rahmen von Maß-, Fertigungs- und Messtoleranzen, gleiche Abbildungseigenschaften werden auch bei anderen azimutalen Lagen erreicht, dann ist jedoch der optische Teil 2 dicker.

In Fig. 19a bis 19k sind Diagramme bezüglich der Parameter analog zu bisherigen Beispielen dargestellt. Bei diesen Ausführungsbeispiel ist eine Fresnellierung mit beispielhaft fünf Zonen ausgebildet. Darüber hinaus ist hier ein azimutaler Versatz zwischen dem Übergang 11 und einem flachen Hauptmeridian 13b ausgebildet, der konkret 1/4 $\pi$ entgegen dem azimutalen Gradienten der Linsenoberfläche beträgt und somit eine Minimierung der Höhe $U_H$ des Übergangs 11 bewirkt.

In Fig. 19k ist wiederum die sagittale Höhe in Abhängigkeit vom Radius gezeigt, wobei hier die Kurve L21 am Windungsende 9 und die Kurve L22 am Windungsanfang 8 dargestellt ist.

Bei diesem Ausführungsbeispiel ist insbesondere gemäß der Tabelle in Fig. 19l die Stufenhöhe $S_H$ der Stufen konstant.

In Fig. 20a bis 20k sind Diagramme bezüglich der Parameter analog zu bisherigen Beispielen dargestellt. Bei diesen Ausführungsbeispiel ist eine Fresnellierung mit beispielhaft fünf Zonen ausgebildet. Darüber hinaus ist hier ein azimutaler Versatz zwischen dem Übergang 11 und einem flachen Hauptmeridian 13b ausgebildet, der konkret 7/4 $\pi$ in Richtung des lokalen Gradienten der Linsenoberfläche beträgt und somit eine Verringerung der Höhe $U_H$ des Übergangs 11 im Vergleich zu einer Ausführung ohne azimutalen Versatz bewirkt.

In Fig. 20k ist wiederum die sagittale Höhe in Abhängigkeit vom Radius gezeigt, wobei hier die Kurve L23 am Windungsende 9 und die Kurve L24 am Windungsanfang 8 dargestellt ist.

Die Beispiele gemäß Fig. 19a bis Fig. 20l können auch mit variabler Stufenhöhe $S_H$ ausgebildet sein.

Bei diesem Ausführungsbeispiel ist insbesondere gemäß der Tabelle in Fig. 19l die Stufenhöhe $S_H$ der Stufen konstant.

**Patentansprüche**

1. Intraokularlinse (1) mit einem optischen Teil (2), der eine in Richtung einer optischen Hauptachse (A) der Intraokularlinse (1) betrachtet erste optische Seite (4) und eine gegenüberliegende zweite optische Seite (5) aufweist, und auf zumindest einer der beiden Seiten (4, 5) eine Helixwindung (6) als Oberflächenstruktur ausgebildet ist, durch welche eine Brechkraft der Intraokularlinse (1) in Umlaufrichtung um die Hauptachse (A) wertmäßig variiert, **dadurch gekennzeichnet, dass** die Intraokularlinse (1) zusätzlich zur Helixwindung (6) ein torisch brechendes Oberflächenprofil (7) aufweist, und das torisch brechende Oberflächenprofil (7) auf zumindest einer Seite (4, 5) ausgebildet ist, wobei zumindest eine Seite (4, 5) des optischen Teils (2) eine in radialer Richtung (r) gestufte Oberflächenkontur aufweist und sich eine Stufenhöhe einer Stufe (35 bis 40) einer ringförmigen Zone (28 bis 34) in azimutaler Richtung um die optische Hauptachse (A) verändert, wobei eine Stufenhöhe in azimutaler Lage eines steileren Hauptmeridians (12a, 12b) des torisch brechenden Oberflächenprofils (7) größer ist, als eine Stufenhöhe in azimutaler Lage eines flacheren Hauptmeridians (13a, 13b) des torisch brechenden Oberflächenprofils (7).

2. Intraokularlinse (1) nach Anspruch 1, **dadurch gekennzeichnet, dass** das torisch brechende Oberflächenprofil (7) und die Helixwindung (6) auf einer gemeinsamen Seite (4, 5) ausgebildet und überlagert sind.

3. Intraokularlinse (1) nach Anspruch 1, **dadurch gekennzeichnet, dass** das torisch brechende Oberflächenprofil (7) auf einer Seite (4, 5) und die Helixwindung (6) auf der anderen Seite (4, 5) ausgebildet sind.

4. Intraokularlinse (1) nach einem der vorhergehenden Ansprüche, **dadurch gekennzeichnet, dass** die Helixwindung (6) eine einmal vollständig um die optische Hauptachse (A) der Intraokularlinse (1) umlaufende Windung ist, und zwischen einem Windungsanfang (8) und einem Windungsende (9) ein sprungartiger Übergang (11) ausgebildet ist.

5. Intraokularlinse (1) nach Anspruch 4, **dadurch gekennzeichnet, dass** die Seite (4, 5), auf welcher zumindest die Helixwindung (6) ausgebildet ist, einen Scheitel (10) aufweist, und ein Differenzwert von einer Scheitelkrümmung (K1) am Windungsanfang (8) und einer Scheitelkrümmung (K2) am Windungsende (9) zwischen 0,001 mm$^{-1}$ und 0,16 mm$^{-1}$ beträgt.

**6.** Intraokularlinse (1) nach Anspruch 4 oder 5, **dadurch gekennzeichnet, dass** die Seite (4, 5), auf welcher zumindest die Helixwindung (6) ausgebildet ist, einen Scheitel (10) aufweist, und ein Differenzwert von einer Scheitelkrümmung (K1) am Windungsanfang (8) und einer Scheitelkrümmung (K2) am Windungsende (9) unabhängig von einer Brechkraft (D1) am Windungsanfang (8) ist.

**7.** Intraokularlinse (1) nach Anspruch 6, **dadurch gekennzeichnet, dass** der Differenzwert der Scheitelkrümmungen (K1, K2) unabhängig von der Brechkraft (D1) am Windungsanfang (8) konstant ist.

**8.** Intraokularlinse (1) nach Anspruch 6 oder 7, **dadurch gekennzeichnet, dass** der Differenzwert der Scheitelkrümmungen (K1, K2) abhängig von der Differenz der Brechkräfte (D1, D2) am Windungsanfang (8) und am Windungsende (9) variiert, insbesondere für eine Differenz der Brechkräfte (D1, D2) zwischen 1 dpt und 10 dpt zwischen 0,015 mm$^{-1}$ und 0,085 mm$^{-1}$ variiert.

**9.** Intraokularlinse (1) nach einem der Ansprüche 4 bis 8, **dadurch gekennzeichnet, dass** der sprungartige Übergang (11) in azimutaler Lage um die optische Hauptachse (A) zu einem Hauptmeridian (13a, 13b) des torisch brechenden Oberflächenprofils (7) versetzt ausgebildet ist, insbesondere in einem azimutalen Winkel zwischen 20° und 70°, insbesondere zwischen 40° und 50°, oder zwischen 200° und 250°, insbesondere zwischen 220° und 230°, zu einem flachen Hauptmeridian ausgebildet ist.

**10.** Intraokularlinse (1) nach einem der vorhergehenden Ansprüche, **dadurch gekennzeichnet, dass** sich eine durch die Helixwindung (6) bewirkte azimutale Brechkraft um die optische Hauptachse (A) zwischen einem Windungsanfang (8) und einem Windungsende (9) nicht-linear verändert.

**11.** Intraokularlinse (1) nach Anspruch 10, **dadurch gekennzeichnet, dass** der Verlauf der durch die Helixwindung (6) bewirkten azimutalen Brechkraft in einem ersten azimutalen Winkelintervall, welches mit dem Windungsanfang (8) beginnt, und einem zweiten azimutalen Winkelintervall, welches mit dem Windungsende (9) endet, flacher ist, als in einem zwischen den beiden Winkelintervallen liegenden dritten Winkelintervall.

**12.** Intraokularlinse (1) nacheinem der vorhergehenden Ansprüche, **dadurch gekennzeichnet, dass** die Stufen (19 bis 22; 35 bis 40) zwischen um die optische Hauptachse (A) umlaufenden und aneinander angrenzenden ringförmige Zonen (14 bis 18; 28 bis 34) gebildet sind, und insbesondere die Oberflächen der ringförmigen Zonen (14 bis 19; 28 bis 34) gleich groß sind.

**13.** Intraokularlinse (1) nacheinem der vorhergehenden Ansprüche, **dadurch gekennzeichnet, dass** eine Stufenhöhe einer Stufe (19 bis 22) einer ringförmigen Zone (14 bis 18) in azimutaler Richtung um die optische Hauptachse (A) konstant ist.

**14.** Intraokularlinse (1) nach einem der vorhergehenden Ansprüche, **dadurch gekennzeichnet, dass** das radial gestufte Oberflächenprofil durch um die optische Hauptachse (A) umlaufende ringförmige Zonen (14 bis 18; 28 bis 34) gebildet ist und Scheitelbrechkräfte von Stufenscheiteln (23 bis 26; 41 bis 46) der Stufen (19 bis 22; 35 bis 40) der ringförmigen Zonen (14 bis 18; 28 bis 34) gleich sind.

**Claims**

**1.** Intraocular lens (1) comprising an optical part (2), which has a first optical side (4) and an opposite second optical side (5) viewed in the direction of a main optical axis (A) of the intraocular lens (1), a helix winding (6) being formed on at least one of the two sides (4, 5) as a surface structure, as a result of which a refractive power of the intraocular lens (1) varies in terms of value in the encircling direction around the main axis (A), **characterized in that** the intraocular lens (1) has a toric refractive surface profile (7) in addition to the helix winding (6) and the toric refractive surface profile (7) is formed on at least one side (4, 5), wherein at least one side (4, 5) of the optical part (2) has a surface contour that is stepped in the radial direction (r) and a step height of a step (35 to 40) of a ring-shaped zone (28 to 34) changes in the azimuthal direction around the main optical axis (A), wherein a step height in an azimuthal position of a steeper main meridian (12a, 12b) of the toric refractive surface profile (7) is greater than a step height in an azimuthal position of a flat main meridian (13a, 13b) of the toric refractive surface profile (7).

**2.** Intraocular lens (1) according to Claim 1, **characterized in that** the toric refractive surface profile (7) and the helix winding (6) are formed and superimposed on a common side (4, 5).

3. Intraocular lens (1) according to Claim 1, **characterized in that** the toric refractive surface profile (7) is formed on one side (4, 5) and the helix winding (6) is formed on the other side (4, 5).

4. Intraocular lens (1) according to any one of the preceding claims, **characterized in that** the helix winding (6) is a winding completely encircling the main optical axis (A) of the intraocular lens (1) once and a jump-like transition (11) is formed between a winding start (8) and a winding end (9).

5. Intraocular lens (1) according to Claim 4, **characterized in that** the side (4, 5) on which at least the helix winding (6) is formed has an apex (10) and the difference value between an apex curvature value (K1) at the winding start (8) and an apex curvature value (K2) at the winding end (9) is between 0.001 mm$^{-1}$ and 0.16 mm$^{-1}$.

6. Intraocular lens (1) according to Claim 4 or 5, **characterized in that** the side (4, 5) in which at least one helix winding (6) is formed has an apex (10) and the difference value between an apex curvature value (K1) at the winding start (8) and an apex curvature value (K2) at the winding end (9) is independent of a refractive power (D1) at the winding start (8).

7. Intraocular lens (1) according to Claim 6, **characterized in that** the difference value of the apex curvature values (K1, K2) is constant, independently of the refractive power (D1) at the winding start (8)

8. Intraocular lens (1) according to Claim 6 or 7, **characterized in that** the difference value between the apex curvature values (K1, K2) varies depending on the difference between the refractive powers (D1, D2) at the winding start (8) and at the winding end (9), in particular varies between 0.015 mm$^{-1}$ and 0.085 mm$^{-1}$ for refractive power (D1, D2) difference between 1 dpt and 10 dpt.

9. Intraocular lens (1) according to any one of Claims 4 to 8, **characterized in that** the jump-like transition (11) in azimuthal position around the main optical axis (A) is formed offset in azimuthal direction with respect to a main meridian (13a, 13b) of the toric refractive surface profile (7), in particular is formed at an azimuthal angle between 20° and 70°, in particular between 40° and 50°, or between 200° and 250°, in particular between 220° and 230°, to a flat main meridian.

10. Intraocular lens (1) according to any one of the preceding claims, **characterized in that** an azimuthal refractive power brought about by the helix winding (6) changes in non-linear fashion around the main optical axis (A) between a winding start (8) and a winding end (9) .

11. Intraocular lens (1) according to Claim 10, **characterized in that** the profile of the azimuthal refractive power brought about by the helix winding (6) is flatter in a first azimuthal angle interval, which starts at the winding start (8), and in a second azimuthal angle interval, which ends with the winding end (9), than in a third angle interval which lies between the two angle intervals.

12. Intraocular lens (1) according to any one of the preceding claims, **characterized in that** the steps (19 to 22; 35 to 40) are formed between adjoining ring-shaped zones (14 to 18; 28 to 34) which encircle the main optical axis (A) and in particular the surfaces of the ring-shaped zones (14 to 19; 28 to 34) have the same size.

13. Intraocular lens (1) according to any one of the preceding claims, **characterized in that** a step height of a step (19 to 22) of a ring-shaped zone (14 to 18) is constant in the azimuthal direction around the main optical axis (A).

14. Intraocular lens (1) according to any one of the preceding claims, **characterized in that** the radially step surface profile is formed by ring-shaped zones (14 to 18; 28 to 34) extending around the main optical axis (A) and vertex powers of step vertices (23 to 26; 41 to 46) of the steps (19 to 22; 35 to 40) of the ring-shaped zones (14 to 18; 28 to 34) are the same.

**Revendications**

1. Lentille intraoculaire (1), comprenant une partie optique (2) qui présente une première face optique (4) et une deuxième face optique opposée (5), vues dans la direction d'un axe principal optique (A) de la lentille intraoculaire (1), et sur au moins l'une des deux faces (4, 5), une spire hélicoïdale (6) est réalisée en tant que structure de surface par laquelle un pouvoir réfringent de la lentille intraoculaire (1) varie en valeur dans la direction périphérique autour de l'axe principal (A), **caractérisée en ce que** la lentille intraoculaire (1) présente en plus de la spire hélicoïdale (6) un profil de surface à réfraction torique (7), et le profil de surface à réfraction torique (7) est réalisé sur au moins une face (4, 5), dans laquelle au moins une face (4, 5) de la partie optique (2) présente dans la direction radiale (r) un contour de surface échelonné et une hauteur d'échelon d'un échelon (35 à 40) d'une zone annulaire (28 à 34) varie dans la direction azimutale autour de l'axe principal optique (A), une hauteur d'échelon dans une position azimutale d'un méridien principal plus pentu (12a, 12b) du profil de surface à réfraction torique (7) étant supérieure à une hauteur d'échelon dans une position azimutale d'un méridien principal plus plat (13a, 13b) du profil de surface à réfraction torique (7) .

2. Lentille intraoculaire (1) selon la revendication 1, **caractérisée en ce que** le profil de surface à réfraction torique (7) et la spire hélicoïdale (6) sont réalisés et superposés sur une face commune (4, 5).

3. Lentille intraoculaire (1) selon la revendication 1, **caractérisée en ce que** le profil de surface à réfraction torique (7) est réalisé sur une face (4, 5) et la spire hélicoïdale (6) est réalisée sur l'autre face (4, 5).

4. Lentille intraoculaire (1) selon l'une quelconque des revendications précédentes, **caractérisée en ce que** la spire hélicoïdale (6) est une spire effectuant un tour complet autour de l'axe principal optique (A) de la lentille intraoculaire (1), et une transition nette (11) est réalisée entre un début de spire (8) et une fin de spire (9).

5. Lentille intraoculaire (1) selon la revendication 4, **caractérisée en ce que** la face (4, 5) sur laquelle est réalisée au moins la spire hélicoïdale (6) présente un sommet (10), et une valeur de différence d'une courbure de sommet (K1) au début de spire (8) et une courbure de sommet (K2) à la fin de spire (9) mesure entre 0,001 mm$^{-1}$ et 0,16 mm$^{-1}$.

6. Lentille intraoculaire (1) selon la revendication 4 ou 5, **caractérisée en ce que** la face (4, 5) sur laquelle est réalisée au moins la spire hélicoïdale (6) présente un sommet (10), et une valeur de différence d'une courbure de sommet (K1) au début de spire (8) et une courbure de sommet (K2) à la fin de spire (9) est indépendante d'un pouvoir réfringent (D1) au début de spire (8).

7. Lentille intraoculaire (1) selon la revendication 6, **caractérisée en ce que** la valeur de différence des courbures de sommet (K1, K2) est constante, indépendamment du pouvoir réfringent (D1) au début de spire (8).

8. Lentille intraoculaire (1) selon la revendication 6 ou 7, **caractérisée en ce que** la valeur de différence des courbures de sommet (K1, K2) varie entre 0,015 mm$^{-1}$ et 0,085 mm$^{-1}$ en fonction de la différence des pouvoirs réfringents (D1, D2) au début de spire (8) et à la fin de spire (9), en particulier pour une différence des pouvoirs réfringents (D1, D2) entre 1 dpt et 10 dpt.

9. Lentille intraoculaire (1) selon l'une quelconque des revendications 4 à 8, **caractérisée en ce que** la transition nette (11) dans la position azimutale autour de l'axe principal optique (A) est réalisée de manière décalée par rapport à un méridien principal (13a, 13b) du profil de surface à réfraction torique (7), en particulier réalisée selon un angle azimutal compris entre 20° et 70°, en particulier entre 40° et 50°, ou entre 200° et 250°, en particulier entre 220° et 230°, pour former un méridien principal plat.

10. Lentille intraoculaire (1) selon l'une quelconque des revendications précédentes, **caractérisée en ce qu'**un pouvoir réfringent azimutal provoqué par la spire hélicoïdale (6) autour de l'axe principal optique (A) varie de manière non linéaire entre un début de spire (8) et une fin de spire (9).

11. Lentille intraoculaire (1) selon la revendication 10, **caractérisée en ce que** la courbe du pouvoir réfringent azimutal provoqué par la spire hélicoïdale (6) dans un premier intervalle d'angle azimutal, qui commence au début de spire (8), et un deuxième intervalle d'angle azimutal, qui se termine à la fin de spire (9), est plus plate que dans un troisième intervalle d'angle situé entre les deux intervalles d'angle.

12. Lentille intraoculaire (1) selon l'une quelconque des revendications précédentes, **caractérisée en ce que** les échelons (19 à 22 ; 35 à 40) sont formés entre des zones annulaires (14 à 18 ; 28 à 34) tournant autour de l'axe principal optique (A) et adjacentes les unes aux autres, et en particulier les surfaces des zones annulaires (14 à 19 ; 28 à 34) sont de grandeur identique.

13. Lentille intraoculaire (1) selon l'une quelconque des revendications précédentes, **caractérisée en ce qu'**une hauteur d'échelon d'un échelon (19 à 22) d'une zone annulaire (14 à 18) est constante dans la direction azimutale autour de l'axe principal optique (A) .

14. Lentille intraoculaire (1) selon l'une quelconque des revendications précédentes, **caractérisée en ce que** le profil de surface radialement échelonné est formé par des zones annulaires (14 à 18 ; 28 à 34) tournant autour de l'axe principal optique (A), et les pouvoirs réfringents de sommet des sommets d'échelon (23 à 26 ; 41 à 46) des échelons (19 à 22 ; 35 à 40) des zones annulaires (14 à 18 ; 28 à 34) sont identiques.

Fig.1a

Fig.1b

Fig.2

F_Sphäre = 20. D    F_Cylinder = 12. D
F_EdoF   = 1. D     F_HOA      = 0. D
CTVA     = 1 mm     rmax       = 3 mm
nmed     = 1.336    niol       = 1.46

Fig.3a

Fig.3b

Fig.3c

Fig.3d

Fig.3e

Fig.3f

Fig.3g

Fig.3h

Fig.3i

Fig.3j

Fig.3k

```
F_Sphäre  = 20. D        F_Cylinder = 12. D
F_EdoF    = 2. D         F_HOA      = 0. D
CTVA      = 1 mm         rmax       = 3 mm
nmed      = 1.336        niol       = 1.46
```

F_Ges

Fig.4a

F_Cyl (φ)

Fig.4b

F_EDoF (φ)

Fig.4c

F_Sphäre

Fig.4d

F_HOA1

Fig.4e

F_HOA2

Fig.4f

Fig.4g

Fig.4h

Fig.4i

Fig.4j

Fig.4k

```
F_Sphäre  = 20. D        F_Cylinder = 12. D
F_EDoF    = 3. D         F_HOA      = 0. D
CTVA      = 1 mm         rmax       = 3 mm
nmed      = 1.336        niol       = 1.46
```

F_Ges

Fig.5a

F_Cyl ($\phi$)

Fig.5b

F_EDoF ($\phi$)

Fig.5c

F_Sphäre

Fig.5d

F_HOA1

Fig.5e

F_HOA2

Fig.5f

Fig.5g

Fig.5h

Fig.5i

Fig.5j

Fig.5k

| Fsph [D] | Fcyl [D] | Fedof [D] | D1 [D] | D2 [D] | r1 [mm] | K1 [mm⁻¹] | Q1 [none] | r2 [mm] | K2 [mm⁻¹] | Q2 [none] | rb [mm] | Qb [none] | ΔK [mm⁻¹] |
|---|---|---|---|---|---|---|---|---|---|---|---|---|---|
| 10 | 6 | 2 | 9. | 11.0003 | 49.5417 | 0.020185 | -7.39923 | 27.481 | 0.0363888 | -5.49404 | -19.0528 | -4.37483 | -0.0162038 |
| 15 | 6 | 2 | 14. | 16.0003 | 24.7628 | 0.0403832 | -3.98004 | 17.6631 | 0.0566151 | -3.3932 | -13.742 | -2.98427 | -0.0162319 |
| 20 | 6 | 2 | 19. | 21.0003 | 16.507 | 0.0605804 | -2.82452 | 13.014 | 0.0768406 | -2.56764 | -10.7383 | -2.37122 | -0.0162602 |
| 25 | 6 | 2 | 24. | 26.0003 | 12.3798 | 0.0807769 | -2.28951 | 10.3023 | 0.0970656 | -2.15073 | -8.80666 | -2.03724 | -0.0162887 |
| 30 | 6 | 2 | 29. | 31.0003 | 9.90362 | 0.100973 | -1.98789 | 8.52584 | 0.11729 | -1.90138 | -7.46005 | -1.82921 | -0.0163173 |
| 35 | 6 | 2 | 34. | 36.0003 | 8.25291 | 0.121169 | -1.79818 | 7.2719 | 0.137516 | -1.74662 | -6.46767 | -1.7108 | -0.0163462 |

| Fsph [D] | Fcyl [D] | Fedof [D] | D1 [D] | D2 [D] | r1 [mm] | K1 [mm⁻¹] | Q1 [none] | r2 [mm] | K2 [mm⁻¹] | Q2 [none] | rb [mm] | Qb [none] | ΔK [mm⁻¹] |
|---|---|---|---|---|---|---|---|---|---|---|---|---|---|
| 10 | 12 | 1 | 9.5 | 10.5006 | 83.1153 | 0.0120315 | -6.81285 | 49.6384 | 0.0201457 | -5.87335 | -15.4685 | -3.39328 | -0.00811422 |
| 15 | 12 | 1 | 14.5 | 15.5006 | 31.0619 | 0.0321938 | -3.81138 | 24.8003 | 0.0403221 | -3.51964 | -11.7683 | -2.56767 | -0.00812833 |
| 20 * | 12 | 1 | 19.5 | 20.5006 | 19.1004 | 0.052355 | -2.75327 | 16.5296 | 0.0604976 | -2.62522 | -9.49022 | -2.15075 | -0.00814254 |
| 25 | 12 | 1 | 24.5 | 25.5006 | 13.7901 | 0.0725156 | -2.25199 | 12.3958 | 0.0806724 | -2.18272 | -7.94659 | -1.9014 | -0.00815685 |
| 30 | 12 | 1 | 29.5 | 30.5006 | 10.7903 | 0.0926758 | -1.96482 | 9.91601 | 0.100847 | -1.92159 | -6.83155 | -1.74663 | -0.00817126 |
| 35 | 12 | 1 | 34.5 | 35.5006 | 8.86241 | 0.112836 | -1.78395 | 8.26297 | 0.121022 | -1.75813 | -5.98838 | -1.6959 | -0.00818577 |

| Fsph [D] | Fcyl [D] | Fedof [D] | D1 [D] | D2 [D] | r1 [mm] | K1 [mm⁻¹] | Q1 [none] | r2 [mm] | K2 [mm⁻¹] | Q2 [none] | rb [mm] | Qb [none] | ΔK [mm⁻¹] |
|---|---|---|---|---|---|---|---|---|---|---|---|---|---|
| 10 | 12 | 2 | 9. | 11.0006 | 125.361 | 0.00797697 | -7.39923 | 41.322 | 0.0242002 | -5.49381 | -15.4685 | -3.39328 | -0.0162232 |
| 15 | 12 | 2 | 14. | 16.0006 | 35.5464 | 0.0281322 | -3.98004 | 22.5308 | 0.0443837 | -3.39312 | -11.7683 | -2.56767 | -0.0162514 |
| 20 * | 12 | 2 | 19. | 21.0006 | 20.7098 | 0.0482864 | -2.82452 | 15.488 | 0.0645662 | -2.5676 | -9.49022 | -2.15075 | -0.0162799 |
| 25 | 12 | 2 | 24. | 26.0006 | 14.6114 | 0.0684398 | -2.28951 | 11.7997 | 0.0847482 | -2.15071 | -7.94659 | -1.9014 | -0.0163085 |
| 30 | 12 | 2 | 29. | 31.0006 | 11.2876 | 0.0885928 | -1.98789 | 9.53016 | 0.10493 | -1.90137 | -6.83155 | -1.74663 | -0.0163373 |
| 35 | 12 | 2 | 34. | 36.0006 | 9.19576 | 0.108746 | -1.79818 | 7.99283 | 0.125112 | -1.74661 | -5.98838 | -1.6959 | -0.0163663 |

| Fsph [D] | Fcyl [D] | Fedof [D] | D1 [D] | D2 [D] | r1 [mm] | K1 [mm⁻¹] | Q1 [none] | r2 [mm] | K2 [mm⁻¹] | Q2 [none] | rb [mm] | Qb [none] | ΔK [mm⁻¹] |
|---|---|---|---|---|---|---|---|---|---|---|---|---|---|
| 10 | 12 | 3 | 8.5 | 11.5006 | 254.943 | 0.00392245 | -8.06831 | 35.3923 | 0.0282547 | -5.16091 | -15.4685 | -3.39328 | -0.0243322 |
| 15 | 12 | 3 | 13.5 | 16.5006 | 41.5444 | 0.0240706 | -4.16694 | 20.6419 | 0.0484452 | -3.27746 | -11.7683 | -2.56767 | -0.0243746 |
| 20 * | 12 | 3 | 18.5 | 21.5006 | 22.6154 | 0.0442177 | -2.90132 | 14.5699 | 0.0686349 | -2.51371 | -9.49022 | -2.15075 | -0.0244172 |
| 25 | 12 | 3 | 23.5 | 26.5006 | 15.5367 | 0.0643639 | -2.32919 | 11.2582 | 0.088824 | -2.12026 | -7.94659 | -1.9014 | -0.0244601 |
| 30 | 12 | 3 | 28.5 | 31.5006 | 11.833 | 0.0845098 | -2.01201 | 9.17321 | 0.109013 | -1.88204 | -6.83155 | -1.74663 | -0.0245033 |
| 35 | 12 | 3 | 33.5 | 36.5006 | 9.55516 | 0.104656 | -1.81327 | 7.7398 | 0.129202 | -1.73607 | -5.98838 | -1.6959 | -0.0245468 |

Fig.6

Fig.7

EP 3 033 648 B1

F_Sphäre = 20. D          F_Cylinder = 12. D
F_EdoF   = 1. D           F_HOA      = 0. D
CTVA     = 1 mm           rmax       = 3 mm
nmed     = 1.336          niol       = 1.46
Fr_steps = 5

**F_Ges**

Fig.8a

**F_Cyl (φ)**

Fig.8b

**F_EDoF (φ)**

Fig.8c

**F_Sphäre**

Fig.8d

**F_HOA1**

Fig.8e

**F_HOA2**

Fig.8f

Fig.8g

Fig.8h

Fig.8i

Fig.8j

Fig.8k

| Zone<br>Index | rmax<br>[mm] | r1<br>[mm] | r2<br>[mm] | K1<br>[mm⁻¹] | K2<br>[mm⁻¹] | ΔK<br>[mm⁻¹] | $S_H$ [μm] |
|---|---|---|---|---|---|---|---|
| 1 | 1.34164 | 19.1004 | 16.5304 | 0.052355 | 0.0604945 | 0.00813945 | -20. |
| 2 | 1.89737 | 19.1021 | 16.5321 | 0.0523504 | 0.0604883 | 0.00813789 | -20. |
| 3 | 2.32379 | 19.1038 | 16.5338 | 0.0523457 | 0.060482 | 0.00813633 | -20. |
| 4 | 2.68328 | 19.1055 | 16.5355 | 0.0523411 | 0.0604758 | 0.00813477 | -20. |
| 5 | 3. | 19.1072 | 16.5372 | 0.0523364 | 0.0604696 | 0.00813321 | -20. |

Fig.8l

```
F_Sphäre  = 20. D        F_Cylinder = 12. D
F_EdoF    = 2. D         F_HOA      = 0. D
CTVA      = 1 mm         rmax       = 3 mm
nmed      = 1.336        niol       = 1.46
Fr_steps  = 5
```

Fig.9a

Fig.9b

Fig.9c

Fig.9d

Fig.9e

Fig.9f

Fig.9g

Fig.9h

Fig.9i

Fig.9j

Fig.9k

| Zone Index | rmax [mm] | r1 [mm] | r2 [mm] | K1 [mm$^{-1}$] | K2 [mm$^{-1}$] | $\Delta$K [mm$^{-1}$] | $S_H$ [μm] |
|---|---|---|---|---|---|---|---|
| 1 | 1.34164 | 20.7098 | 15.4887 | 0.0482864 | 0.0645631 | 0.0162768 | -20. |
| 2 | 1.89737 | 20.7115 | 15.4904 | 0.0482824 | 0.0645561 | 0.0162737 | -20. |
| 3 | 2.32379 | 20.7132 | 15.4921 | 0.0482784 | 0.064549 | 0.0162705 | -20. |
| 4 | 2.68328 | 20.7149 | 15.4938 | 0.0482745 | 0.0645419 | 0.0162674 | -20. |
| 5 | 3. | 20.7166 | 15.4955 | 0.0482705 | 0.0645348 | 0.0162643 | -20. |

Fig.9l

```
F_Sphäre  = 20. D        F_Cylinder = 12. D
F_EdoF    = 3. D         F_HOA      = 0. D
CTVA      = 1 mm         rmax       = 3 mm
nmed      = 1.336        niol       = 1.46
Fr_steps  = 5
```

F_Ges

Fig.10a

F_Cyl ($\phi$)

Fig.10b

F_EDoF ($\phi$)

Fig.10c

F_Sphäre

Fig.10d

F_HOA1

Fig.10e

F_HOA2

Fig.10f

Fig.10g

Fig.10h

Fig.10i

Fig.10j

Fig.10k

| Zone Index | rmax [mm] | r1 [mm] | r2 [mm] | K1 [mm$^{-1}$] | K2 [mm$^{-1}$] | ΔK [mm$^{-1}$] | $S_H$ [μm] |
|---|---|---|---|---|---|---|---|
| 1 | 1.34164 | 22.6154 | 14.5705 | 0.0442177 | 0.0686318 | 0.0244141 | -20. |
| 2 | 1.89737 | 22.6171 | 14.5722 | 0.0442144 | 0.0686238 | 0.0244094 | -20. |
| 3 | 2.32379 | 22.6188 | 14.5739 | 0.044211 | 0.0686158 | 0.0244047 | -20. |
| 4 | 2.68328 | 22.6205 | 14.5756 | 0.0442077 | 0.0686078 | 0.0244001 | -20. |
| 5 | 3. | 22.6222 | 14.5773 | 0.0442044 | 0.0685998 | 0.0243954 | -20. |

Fig.10l

Fig.11

```
F_Sphäre  = 20. D        F_Cylinder = 6. D
F_EdoF    = 1. D         F_HOA      = 0. D
CTVA      = 1. mm        rmax       = 3 mm
nmed      = 1.336        niol       = 1.46
rtarget   = -30 mm       Qtarget    = -2.5
Fr_steps  = 7
```

Fig.12a

Fig.12b

Fig.12c

Fig.12d

Fig.12e

Fig.12f

Fig.12g

Fig.12h

Fig.12i

Fig.12j

Fig.12k

| Zone Index | rmax [mm] | r1 [mm] | r2 [mm] | K1 [mm⁻¹] | K2 [mm⁻¹] | ΔK [mm⁻¹] | S_H [μm] |
|---|---|---|---|---|---|---|---|
| 1 | 1.13389 | 15.5923 | 13.8505 | 0.064134 | 0.0721996 | 0.00806562 | -24.855 |
| 2 | 1.60357 | 15.594 | 13.8522 | 0.064127 | 0.0721908 | 0.00806375 | -24.6225 |
| 3 | 1.96396 | 15.5957 | 13.8539 | 0.0641201 | 0.0721819 | 0.00806188 | -24.3956 |
| 4 | 2.26779 | 15.5974 | 13.8556 | 0.0641131 | 0.0721731 | 0.00806002 | -24.1732 |
| 5 | 2.53546 | 15.5991 | 13.8573 | 0.0641061 | 0.0721642 | 0.00805815 | -23.9552 |
| 6 | 2.77746 | 15.6008 | 13.859 | 0.0640991 | 0.0721554 | 0.00805629 | -23.7411 |
| 7 | 3. | 15.6025 | 13.8607 | 0.0640921 | 0.0721466 | 0.00805442 | -23.5308 |

Fig.12l

F_Sphäre = 20. D      F_Cylinder = 6. D
F_EdoF = 2. D      F_HOA = 0. D
CTVA = 1. mm      rmax = 3 mm
nmed = 1.336      niol = 1.46
rtarget = -30 mm      Qtarget = -2.5
Fr_steps = 7

Fig.13a

Fig.13b

Fig.13c

Fig.13d

Fig.13e

Fig.13f

Fig.13g

Fig.13h

Fig.13i

Fig.13j

Fig.13k

| Zone Index | rmax [mm] | r1 [mm] | r2 [mm] | K1 [mm⁻¹] | K2 [mm⁻¹] | ΔK [mm⁻¹] | $S_{H[\mu m]}$ |
|---|---|---|---|---|---|---|---|
| 1 | 1.13389 | 16.6385 | 13.1179 | 0.0601017 | 0.0762319 | 0.0161302 | -27.4293 |
| 2 | 1.60357 | 16.6402 | 13.1196 | 0.0600956 | 0.0762221 | 0.0161265 | -27.1631 |
| 3 | 1.96396 | 16.6419 | 13.1213 | 0.0600895 | 0.0762122 | 0.0161227 | -26.9035 |
| 4 | 2.26779 | 16.6435 | 13.123 | 0.0600833 | 0.0762023 | 0.016119 | -26.6493 |
| 5 | 2.53546 | 16.6452 | 13.1247 | 0.0600772 | 0.0761925 | 0.0161153 | -26.4002 |
| 6 | 2.77746 | 16.6469 | 13.1264 | 0.0600711 | 0.0761826 | 0.0161115 | -26.1559 |
| 7 | 3. | 16.6486 | 13.1281 | 0.0600649 | 0.0761727 | 0.0161078 | -25.9162 |

Fig.13l

39

```
F_Sphäre  = 20. D        F_Cylinder = 6. D
F_EdoF    = 3. D         F_HOA      = 0. D
CTVA      = 1. mm        rmax       = 3 mm
nmed      = 1.336        niol       = 1.46
rtarget   = -30 mm       Qtarget    = -2.5
Fr_steps  = 7
```

F_Ges

Fig.14a

F_Cyl (φ)

Fig.14b

F_EDoF (φ)

Fig.14c

F_Sphäre

Fig.14d

F_HOA1

Fig.14e

F_HOA2

Fig.14f

Fig.14g

Fig.14h

Fig.14i

Fig.14j

Fig.14k

| Zone Index | rmax [mm] | r1 [mm] | r2 [mm] | K1 [mm⁻¹] | K2 [mm⁻¹] | ΔK [mm⁻¹] | $S_{H[\mu m]}$ |
|---|---|---|---|---|---|---|---|
| 1 | 1.13389 | 17.835 | 12.4589 | 0.0560694 | 0.0802642 | 0.0241948 | -30.0025 |
| 2 | 1.60357 | 17.8367 | 12.4606 | 0.0560641 | 0.0802533 | 0.0241892 | -29.7005 |
| 3 | 1.96396 | 17.8384 | 12.4623 | 0.0560588 | 0.0802423 | 0.0241836 | -29.4061 |
| 4 | 2.26779 | 17.8401 | 12.4639 | 0.0560534 | 0.0802314 | 0.024178 | -29.1182 |
| 5 | 2.53546 | 17.8418 | 12.4656 | 0.0560481 | 0.0802205 | 0.0241724 | -28.8363 |
| 6 | 2.77746 | 17.8435 | 12.4673 | 0.0560428 | 0.0802095 | 0.0241668 | -28.5601 |
| 7 | 3. | 17.8452 | 12.469 | 0.0560374 | 0.0801986 | 0.0241612 | -28.2893 |

Fig.14l

```
F_Sphäre  = 20. D        F_Cylinder = 6. D
F_EdoF    = 3. D         F_HOA      = 0. D
CTVA      = 1. mm        rmax       = 3 mm
nmed      = 1.336        niol       = 1.46
rtarget   = -30 mm       Qtarget    = -2.5
Fr_steps  = 7
```

F_Ges

Fig.15a

F_Cyl ($\phi$)

Fig.15b

F_EDoF ($\phi$)

Fig.15c

F_Sphäre

Fig.15d

F_HOA1

Fig.15e

F_HOA2

Fig.15f

Fig.15g

Fig.15h

Fig.15i

Fig.15j

Fig.15k

| Zone Index | rmax [mm] | r1 [mm] | r2 [mm] | K1 [mm$^{-1}$] | K2 [mm$^{-1}$] | $\Delta$K [mm$^{-1}$] | $S_{H}$ [µm] |
|---|---|---|---|---|---|---|---|
| 1 | 1.13389 | 17.835 | 12.4589 | 0.0560694 | 0.0802642 | 0.0241948 | -15.4299 |
| 2 | 1.60357 | 17.8367 | 12.4606 | 0.0560641 | 0.0802533 | 0.0241892 | -15.2003 |
| 3 | 1.96396 | 17.8384 | 12.4623 | 0.0560588 | 0.0802423 | 0.0241836 | -14.9497 |
| 4 | 2.26779 | 17.8401 | 12.4639 | 0.0560534 | 0.0802314 | 0.024178 | -14.6776 |
| 5 | 2.53546 | 17.8418 | 12.4656 | 0.0560481 | 0.0802205 | 0.0241724 | -14.3841 |
| 6 | 2.77746 | 17.8435 | 12.4673 | 0.0560428 | 0.0802095 | 0.0241668 | -14.0693 |
| 7 | 3. | 17.8452 | 12.469 | 0.0560374 | 0.0801986 | 0.0241612 | -13.7337 |

Fig.15l

```
F_Sphäre  = 20. D        F_Cylinder = 2. D
F_EdoF    = 1.5 D        F_HOA      = 0.5 D
CTVA      = 1 mm         rmax       = 3 mm
nmed      = 1.336        niol       = 1.46
Fr_steps  = 1
```

F_Ges

Fig.16a

F_Cyl ($\phi$)

Fig.16b

F_EDoF ($\phi$)

Fig.16c

F_Sphäre

Fig.16d

F_HOA1

Fig.16e

F_HOA2

Fig.16f

Fig.16g

Fig.16h

Fig.16i

Fig.16j

Fig.16k

F_Sphäre = 20. D       F_Cylinder = 2. D
F_EdoF   = 1.5 D        F_HOA     = 0.5 D
CTVA     = 1 mm        rmax      = 3 mm
nmed     = 1.336       niol      = 1.46
Fr_steps = 5

Fig.17a

Fig.17b

Fig.17c

Fig.17d

Fig.17e

Fig.17f

46

Fig.17g

Fig.17h

Fig.17i

Fig.17j

Fig.17k

| Zone Index | rmax [mm] | r1 [mm] | r2 [mm] | K1 [mm$^{-1}$] | K2 [mm$^{-1}$] | ΔK [mm$^{-1}$] | $S_H$ [μm] |
|---|---|---|---|---|---|---|---|
| 1 | 1.34164 | 14.1285 | 12.0534 | 0.0707787 | 0.0829643 | 0.0121856 | -20. |
| 2 | 1.89737 | 14.1302 | 12.0551 | 0.0707702 | 0.0829526 | 0.0121824 | -20. |
| 3 | 2.32379 | 14.1319 | 12.0568 | 0.0707617 | 0.0829409 | 0.0121793 | -20. |
| 4 | 2.68328 | 14.1336 | 12.0585 | 0.0707532 | 0.0829293 | 0.0121761 | -20. |
| 5 | 3. | 14.1353 | 12.0602 | 0.0707447 | 0.0829176 | 0.0121729 | -20. |

Fig.17l

```
F_Sphäre = 20. D        F_Cylinder = 12. D
F_EdoF   = 2. D         F_HOA      = 0. D
CTVA     = 1 mm         rmax       = 3 mm
nmed     = 1.336        niol       = 1.46
Fr_steps = 5
```

Fig.18

```
F_Sphäre  = 20. D        F_Cylinder = 12. D
F_EdoF    = 2. D         F_HOA      = 0. D
CTVA      = 1 mm         rmax       = 3 mm
nmed      = 1.336        niol       = 1.46
Fr_steps  = 5
Axi Spr.  = 0.785398 rad
```

Fig.19a

Fig.19b

Fig.19c

Fig.19d

Fig.19e

Fig.19f

Fig.19g

Fig.19h

Fig.19i

Fig.19j

Fig.19k

| Zone Index | rmax [mm] | r1 [mm] | r2 [mm] | K1 [mm⁻¹] | K2 [mm⁻¹] | ΔK [mm⁻¹] | S_H [μm] |
|---|---|---|---|---|---|---|---|
| 1 | 1.34164 | 8.9731 | 10.0958 | 0.111444 | 0.0990512 | 0.012393 | -20. |
| 2 | 1.89737 | 8.9748 | 10.0975 | 0.111423 | 0.0990346 | 0.0123885 | -20. |
| 3 | 2.32379 | 8.9765 | 10.0992 | 0.111402 | 0.0990179 | 0.0123841 | -20. |
| 4 | 2.68328 | 8.9782 | 10.1009 | 0.111381 | 0.0990013 | 0.0123797 | -20. |
| 5 | 3. | 8.9799 | 10.1026 | 0.11136 | 0.0989846 | 0.0123752 | -20. |

Fig.19l

```
F_Sphäre  = 20. D          F_Cylinder = 12. D
F_EdoF    = 2. D           F_HOA      = 0. D
CTVA      = 1 mm           rmax       = 3 mm
nmed      = 1.336          niol       = 1.46
Fr_steps  = 5
Axi Spr.  = 5.49779 rad
```

Fig.20a

Fig.20b

Fig.20c

Fig.20d

Fig.20e

Fig.20f

Fig.20g

Fig.20h

Fig.20i

Fig.20j

Fig.20k

| Zone Index | $r_{max}$ [mm] | $r_1$ [mm] | $r_2$ [mm] | $K_1$ [mm$^{-1}$] | K2 [mm$^{-1}$] | $\Delta$K [mm$^{-1}$] | $S_{H}$ [μm] |
|---|---|---|---|---|---|---|---|
| 1 | 1.34164 | 8.67108 | 10.3024 | 0.115326 | 0.0970649 | 0.018261 | -20. |
| 2 | 1.89737 | 8.67278 | 10.3041 | 0.115303 | 0.0970489 | 0.0182544 | -20. |
| 3 | 2.32379 | 8.67448 | 10.3058 | 0.115281 | 0.0970329 | 0.0182478 | -20. |
| 4 | 2.68328 | 8.67618 | 10.3075 | 0.115258 | 0.0970169 | 0.0182413 | -20. |
| 5 | 3. | 8.67787 | 10.3092 | 0.115236 | 0.0970009 | 0.0182347 | -20. |

Fig.20l

**IN DER BESCHREIBUNG AUFGEFÜHRTE DOKUMENTE**

**In der Beschreibung aufgeführte Patentdokumente**

- DE 102011101899 A1
- DE 102011114752 A1
- WO 2011102719 A1